Europäisches Patentamt

European Patent Office

Office européen des brevets

(11) Publication number: **0 388 448 B1**

# EUROPEAN PATENT SPECIFICATION

(45) Date of publication of patent specification: **26.07.95** (51) Int. Cl.⁶: **D21H 25/18**, C07C 29/70

(21) Application number: **89909645.7**

(22) Date of filing: **23.08.89**

(86) International application number:
**PCT/US89/03623**

(87) International publication number:
**WO 90/03466 (05.04.90 90/08)**

(54) **MASS TREATMENT OF CELLULOSIC MATERIALS.**

(30) Priority: **30.09.88 US 252421**

(43) Date of publication of application:
**26.09.90 Bulletin 90/39**

(45) Publication of the grant of the patent:
**26.07.95 Bulletin 95/30**

(84) Designated Contracting States:
**AT BE CH DE FR GB IT LI LU NL SE**

(56) References cited:
**WO-A-85/02176**
**US-A- 3 676 182**
**US-A- 4 318 963**

(73) Proprietor: **LITHIUM CORPORATION OF AMER-ICA**
**449 North Cox Road**
**Gastonia NC 28054 (US)**

(72) Inventor: **KAMIENSKI, Conrad, William**
**516 Eastwood Drive**
**Gastonia, NC 28054 (US)**
Inventor: **WEDINGER, Robert, Scott**
**3200 Eppinette Court**
**Gastonia, NC 28054 (US)**

(74) Representative: **Kooy, Leendert Willem et al**
**OCTROOIBUREAU VRIESENDORP & GAADE**
**P.O. Box 266**
**NL-2501 AW The Hague (NL)**

Rank Xerox (UK) Business Services
(3.10/3.09/3.3.3)

## Description

This invention concerns a method for treating cellulosic materials such as paper by neutralizing the acidity of the paper and buffering the paper to the alkaline side to provide protection of the paper from post-treatment acid attack and to improve paper performance and novel chemical compositions useful in practicing this method of paper treatment.

Paper has been made for nearly 2,000 years; some early papers have lasted half of this time and others made during the middle ages are in excellent condition. Ancient handmade paper was too soft material for receiving ink so it was sized by dipping the sheets in hot animal gelatin or starch. As papermakers shifted to machine made paper, different procedures for sizing the paper were necessary. This problem was solved by "tub sizing," which is done by adding a mixture of alum and rosin to the wet fiber slurry before the sheet was formed on the machine, thereby coating the fibers in the paper with rosin and making the paper suitable for use in printing paper or writing paper. Unfortunately, alum, aluminum sulfate [$Al_2(SO_4)_3$], which is used in tub sizing to uniformly distribute the rosin throughout the sheet is one of the major causes of acid in paper. Subsequent reaction between aluminum sulfate and water produces sulfuric acid. Other sources of acid in the paper, of course, include industrial atmospheres that have sulfur dioxide and nitrogen oxides in the air which form acid in the paper. Thus, most books printed in the last 100 or 150 years were printed on acid paper which becomes brittle and disintegrates in a period of 5-75 years or more depending on the care with which books have been stored.

Almost every library in the world is filled with books made with this acid paper, and therefore, have large collections of books which are disintegrating. This problem of acidity in books has been recognized for quite a long time and a great deal of work has been done to establish a process for deacidifying such books. A recent report, "Mass Deacidification for Libraries" by George Martin Cunha, Adjunct Professor of Conservation, College of Library and Information Science, University of Kentucky, was printed in Library Technology Reports, Volume 23, No. 3, May-June 1987. The Cunha report claims to have reviewed all known experiments with methods of mass deacidification and six were thoroughly investigated. One of the major methods of mass deacidification considered was the so-called diethyl zinc (DEZ) system which appears to be covered by US-A-3,969,459 and US-A-4,051,276 assigned to the United States of America as represented by the Librarian of Congress. Mass deacidification with diethyl zinc is a 50-55 hour three-phase process consisting of preconditioning, permeation and passivation. The DEZ system under development by the Library of Congress will handle thousands of books per cycle. However, liquid diethyl zinc is pyrophoric (will spontaneously ignite when exposed to air) and will react explosively with water. The fire and explosive hazards of diethyl zinc make it a dangerous chemical to work with and probably one that every library could not contemplate using. Moreover, the fire and explosive hazards have caused considerable expensive design problems in developing suitable equipment for this process.

Another major method of mass deacidification evaluated in the Cunha report was one employing methoxymagnesium methylcarbonate in a solution of alcohol and fluorocarbons in treating books in mass. The system is a liquid process designed to dissolve, transport and deposit the chemicals into book pages to neutralize acids present in the paper and to deposit buffering chemicals that will neutralize any acids that may subsequently contaminate the paper. This system appears to be covered by US-4,318,963 of Richard D. Smith and US-A-3,939,091 of George B. Kelly (assigned to the United States of America as represented by the Librarian of Congress) and is based on earlier US-A-3,676,055 and US-A-3,676,182 to Richard D. Smith. The process employs methoxymagnesiummethyl carbonate dissolved in a liquid solution of fluorocarbons and methyl alcohol. This alcohol is necessary to promote solution of the magnesium compound. This solution reacts with water in the paper to form magnesium carbonate, magnesium hydroxide and magnesium oxide, some of which react with the acid in the paper to form neutralized salts. The remaining mixture of carbonate, hydroxide and oxide remains in the paper as basic magnesium carbonate which forms an alkaline reserve or buffering agent that will neutralize future acid contamination of the paper. Disadvantages of the process include feathering of alcohol-soluble inks and colors and attack on some highly nitrated book covers, and thus a pre-sorting by uses is required.

According to the Cunha report, ammonia has been used in India for mass deacidification of books. Langwell in England used cyclohexylamine carbonate, while in the United States the Barrow Laboratory in Virginia conceived the use of morpholine (US-A-3,771,958). The morpholine, ammonia and cyclohexylamine carbonate systems were moderately effective deacidifiers, but did not provide a buffer in the paper to provide protection of the paper from post-treatment acid attack. A patent to R.A. Kundrot (US-A-4,522,843) claims a method of deacidifying books using alkaline particles of a basic metal suspended in an aerosol.

The present invention provides a process in which certain carbonated magnesium alkoxyalkoxides can be readily prepared and dissolved in a wide variety of solvents both liquid and gaseous at ordinary temperatures and said solutions applied to paper, books, and other cellulosic materials to both deacidify, coat them in a protective manner, and to provide them with added strength.

Summary of the Invention

In accordance with this invention, there is provided a process for deacidifying paper using certain magnesium and zinc alkoxyalkoxides, described in US-A-4,634,786, cols. 7-10 and 17, and dissolved in hydrocarbon or halocarbon solvents, which are treated with gaseous carbon dioxide to, unexpectedly, yield solutions possessing a much reduced viscosity. These solutions can be readily solvent-stripped to yield fluid, somewhat viscous liquid residues, which readily redissolve in a variety of gaseous and liquid hydrocarbon and halocarbon solvents to yield highly concentrated, low viscosity solutions, which can be adjusted in concentration to that needed for treatment of books and paper. Optionally an aluminum or zinc alkoxide can be added to these carbonated alkoxide compositions to help solubilize the components of the compositions.

Examination of the resulting carbonated magnesium-containing residues, or solutions thereof, show that approximately 50% of the magnesium alkoxyalkoxide groupings have been converted to magnesium alkoxyalkyl carbonate groupings.

These carbonated magnesium alkoxyalkoxides (or magnesium alkoxyalkyl carbonates) either in a neat form or in a solution form, can be treated with gases, such as gaseous hydrocarbons, for example ethane or carbon dioxide, under relatively low pressure, to yield greatly expanded solutions of the magnesium-containing product. Thus, for example, treatment of a 5-10 weight percent solution of magnesium $\underline{n}$-hexylcarbitolate (carbonated) in hexane with sufficient ethane gas to raise the pressure to about 3.448 MPa (500 psi) causes the liquid volume of the solution to increase to approximately twice its original volume.

The above property of volume expandability can be utilized to alternately cover and uncover paper products suspended in the same chamber. Thus, a strip of paper cut from a book, when suspended vertically above the original carbonated magnesium $\underline{n}$-hexylcarbitolate solution in hexane, is contacted by the solution to a point more than halfway to the top of the paper strip, by pressurizing the magnesium-containing solution with ethane as described above. Release of the pressure and venting of the gas returns the liquid solution to its original volume below the bottom edge of the paper strip.

Said paper strip is now treated evenly with the magnesium-containing compound and possesses a satiny feel.

Unlike magnesium alkoxide carbonates described in the literature, such as methoxymagnesium methyl carbonate no alcohol co-solvent is required to maintain the solubility of the carbonated magnesium alkoxides of this invention either in hydrocarbon or halocarbon solution. Alcohol co-solvents have been shown to be detrimental in book deacidification, dissolving inks and colors and attacking plastic covers in the books.

In addition, other metallic alkoxides (such as aluminum alkoxides) based on alcohols described in US-A-4,634,786, cols. 7-10, and 17 can be prepared in liquid form and dissolved in a variety of solvents, including hydrocarbons and halocarbons. The corresponding aluminum alkoxides can be readily prepared and combined with the carbonated magnesium and/or zinc alkoxides of this invention and dissolved in a variety of solvents to yield solutions which can be gas expanded or used directly in the treatment of paper and books.

Thus, it is one advantage of this invention to make available carbonated magnesium alkoxides possessing a high solubility in liquid hydrocarbon and halocarbon solvents, said solutions also having the property of low viscosity.

It is another advantage of this invention to provide a process for greatly expanding the volume of these solutions of carbonated magnesium alkoxides without causing the separation of carbonated magnesium alkoxides from these solutions.

It is another advantage of this invention to provide a simplified process for the utilization of such expanded solutions of carbonated magnesium alkoxide in the treatment of paper, books, and other cellulosic materials, whereby the latter are deacidified, buffered, and given a permanent finish by these expanded solutions.

It is another advantage of this invention to provide a simplified process for the utilization of solutions of carbonated magnesium alkoxide, per se, in the treatment of paper, books, and other cellulosic materials whereby the latter are deacidified, buffered and strengthened by these solutions.

It is yet another advantage of this invention to provide such deacidifying carbonated magnesium alkoxide solutions in the essential absence of co-solvent alcohols.

EP 0 388 448 B1

It is also an advantage of this invention to provide other low viscosity metallic alkoxides solutions for use as deacidifying solutions in liquid or gaseous hydrocarbon and halocarbon solvents, such as, for example aluminum alkoxides, alone or in combination with the carbonated magnesium and zinc alkoxides of this invention. Accordingly the present invention provides a process for deacidifying cellulosic materials comprising contacting the cellulosic material with a solution in a hydrocarbon or halocarbon or mixtures thereof containing 0.01-1 mole/l of one or more substituted metal alkoxides having the formula

$$X_y M^a (OR)_{a-y} . (R^1 OH)_x \quad (1)$$

wherein:

(I) OR is a group selected from 2-alkoxyalkoxy- and $\omega$-alkoxypolyalkoxy- groups of the formula

$$[-OCH(R^2)CH_2(OCH(R^2)CH_2)_n OR^3]$$

wherein $R^2$ is selected from H and $-CH_3$ and $R^3$ is selected from alkyl groups of 1 to 18 carbon atoms, cycloalkyl groups of 3 to 18 carbon atoms and aryl, arylalkyl and alkylaryl groups of 6 to 18 carbon atoms and n is a value of 1 to 20 and wherein -OR is optionally carbonated;

(II) X is a group selected from

    (a) 2-dialkylaminoalkoxy- and $\omega$-dialkylaminopolyalkoxy groups of the formula

$$[-OCH(R^{2'})CH_2(OCH(R^{2'})CH_2)_m NR^{3'}_2]$$

wherein $R^{2'}$ is selected from hydrogen and methyl, $R^{3'}$ is selected from alkyl groups of 1 to 18 atoms, cycloalkyl groups of 3 to 18 carbon atoms and aryl, arylalkyl and alkylaryl groups of 6 to 18 carbon atoms and m is a value of 1 to 20;

    (b) a halogen selected from chlorine and bromine;

    (c) an organic group $-R^4$ wherein $R^4$ is selected from the group consisting of alkyl groups containing 1 to 18 carbon atoms, cycloalkyl groups containing 3 to 18 carbon atoms and aryl, arylalkyl and alkylaryl groups containing 6 to 18 carbon atoms;

    (d) an acyloxy group of the formula $[-O(O)CR^4]$ wherein $R^4$ has the hereintobefore ascribed meaning;

    (e) alkoxypolyalkoxy- groups of the formula

$$[-OCH(R^{2'})CH_2(OCH(R^{2'})CH_2)_m OR^{3'}]$$

wherein $R^{2'}$, $R^{3'}$ and m have the hereintobefore ascribed meanings;

    (f) an alkoxy group $-OR^4$ wherein $R^4$ has the hereintobefore ascribed meanings;

(III) M is a metal selected from groups IIa and IIb of the Periodic Table and aluminium and mixtures thereof;

(IV) $R^1 OH$ is a compound in which $R^1 O$ is a group selected from

    (a) alkoxy groups of the formula $R^{4'}O$ wherein $R^{4'}$ has the meanings hereintobefore ascribed for $R^4$;

    (b) 2-alkoxyalkoxy- and $\omega$-alkoxypolyalkoxy- groups of the formula

$$[-OCH(R^{2''})CH_2(OCH(R^{2''})CH_2)_o OR^{3''}]$$

wherein $R^{2''}$, $R^{3''}$ and o have the meanings hereintobefore ascribed for $R^2$, $R^3$ and n;

    (c) 2-dialkylaminoalkoxy- and $\omega$-dialkylaminopolyakoxy groups of the formula

$$[-OCH(R^{2''})CH_2(OCH(R^{2''})CH_2)_o NR^{3''}_2]$$

(V) a is the valence of the metal M;

(VI) y has a value between zero and one; and

(VII) x has a value of zero to two;

Preferably, at least one of the compounds used is a compound of formula $X_y M^a(OR)_{a-y}.(R^1OH)_x$ in which either $X_y$ or -OR is carbonated wherein $R^1OH$, M, a, y and x have the hereintobefore ascribed meanings; and

(I) OR is a group selected from 2-alkoxyalkoxy- and $\omega$-alkoxypolyalkoxy groups of the formula

$$[-OCH(R^2)CH_2(OCH(R^2)CH_2)_n OR^3]$$

4

wherein $R^2$ is selected from H and -$CH_3$ and $R^3$ is selected from alkyl groups of 1 to 18 carbon atoms, cycloalkyl groups of 3 to 18 carbon atoms and aryl, arylalkyl and alkylaryl groups of 6 to 18 carbon atoms and n is a value of 1 to 20; and

(II) X is a group selected from

(a) 2-dialkylaminoalkoxy- and $\omega$-dialkylaminopolyalkoxy groups of the formula

$$[-OCH(R^{2'})CH_2(OCH(R^{2'})CH_2)_mNR^{3'}_2]$$

wherein $R^{2'}$ is selected from hydrogen and methyl, $R^{3'}$ is selected from alkyl groups of 1 to 18 carbon atoms, cycloalkyl groups of 3 to 18 carbon atoms and aryl, arylalkyl and alkylaryl groups of 6 to 18 carbon atoms and m is a value of 1 to 20;

(b) an -alkoxypolyalkoxy- group of the formula

$$[-OCH(R^{2'})CH_2(OCH(R^{2'})CH_2)_mOR^{3'}]$$

wherein $R^{2'}$, $R^{3'}$ and m have the hereintobefore ascribed meanings; and,

(c) an alkoxy group -$OR^4$ wherein $R^4$ has the hereintobefore ascribed meaning.

Examples of compounds of the formula

$$X_yM^a(OR)_{a-y}\bullet(R^1OH)_x$$

include but are not limited to

$CH_3OMgOCH_2CH_2OCH_2CH_2OC_2H_5$

$C_6H_{13}OMgOCH(CH_3)CH_2-OCH(CH_3)CH_2OCH_3$

$C_2H_5OZnOCH_2CH_2(OCH_2CH_2)_{6.4}OCH_3\bullet(C_4H_9O-(CH_2CH_2O)_3H)_{0.5}$

$C_3H_7OAl(OCH_2CH_2(OCH_2CH_2)_2OC_4H_9)_2$

$CH_3O(CH_2CH(CH_3)O)_2CH_2CH(CH_3)OMgOCH_2CH_2(OCH_2CH_2)_3OCH_3$

$C_4H_9O(CH_2CH_2O)_2-CH_2CH_2OZnO-CH(CH_3)CH_2OCH(CH_3)CH_2OCH_3\bullet(C_2H_5OH)$

$C_2H_5OCH_2CH_2OAl(OCH_2CH_2OCH_2CH_2OC_4H_9)_2$

$(CH_3)_2N-CH_2CH_2OMgOCH_2CH_2OCH_2CH_2OC_2H_5\bullet C_2H_5OH$

$(C_2H_5)_2NCH_2CH(CH_3)OZnOCH_2CH_2(OCH_2CH_2)_{6.4}OCH_3\bullet (C_2H_5)NCH_2CH(CH_3)OH$

$(CH_3)_2NCH_2CH_2OCH_2CH_2OAl(OCH(CH_3)CH_2(OCH(CH_3)CH_2)_2OCH_3$

$ClMgOCH_2CH_2(OCH_2CH_2)_3OC_4H_9.C_4H_9O(CH_2CH_2O)_3H$

$BrZnOCH(CH_3)CH_2OCH(CH_3)CH_2OCH_3$

$ClAlOCH_2CH_2(OCH_2CH_2)_2OC_4H_9$

$C_2H_5OC(O)OMgOCH_2CH_2OCH_2CH_2OC_4H_9$

$CH_3OC(O)OZnOCH(CH_3)CH_2-O-CH(CH_3)CH_2OCH_3\bullet_{0.5}(C_4H_9O(CH_2CH_2O)_3H)$

$CH_3OC(O)OMgOCH_2CH_2(OCH_2CH_2)OC_2H_5\bullet_{0.5}C_4H_9O(CH_2CH_2O)_3H$

$C_4H_9MgOCH_2CH_2(OCH_2CH_2)_{6.4}OCH_3$

$C_2H_5ZnOCH_2CH_2(OCH_2CH_2)_{6.4}OCH_3$

$C_6H_5Al(OCH_2CH_2(OCH_2CH_2)_2OC_4H_9)_2$

$CH_3CH_2C(O)OMgOCH(CH_3)CH_2OCH(CH_3)CH_2OCH_3\bullet C_2H_5OH$

$C_4H_9C(O)OZnOCH_2CH_2(OCH_2CH_2)_{6.4}OCH_3$

$C_4H_9C(O)OAlOCH(CH_3)CH_2OCH(CH_3)CH_2OCH_3$

Substituted alkoxides, such as zinc or magnesium alkoxides of the above formula (1), wherein II is (e) or (f), dissolved in hydrocarbon or halocarbon solvents are treated, according to this invention, with gaseous carbon dioxide at atmospheric pressure and above, to convert them to carbonated magnesium or zinc alkoxides, as described in US-A-3,939,091, col. 3, which discloses passing carbon dioxide into a solution of magnesium methoxide (8 to 9 weight percent) in methanol until the solution or suspension is saturated with carbon dioxide.

Carbonation of the magnesium or zinc alkoxide solutions can be done at temperatures between about 0° and about 100°C and at pressures between about atmospheric and 6.895 MPa (1000 psi). Then solvents are stripped from the solution under reduced pressure to yield the neat liquid carbonated magnesium or zinc alkoxides (or alkoxymetalalkoxyalkyl carbonates) shown below. Small amounts (up to 2 moles per mole of carbonated metal alkoxide) of alcohols may be present which are complexed (tightly held) with the carbonated metal alkoxide and are beneficial in promoting solubilization of the carbonated metal alkoxide in the solvents used for treatment of the cellulosic materials.

Examples of the metal alkoxides which can be carbonated with carbon dioxide according to this invention are magnesium and zinc bis-2-alkoxyalkoxides and magnesium and zinc bis-omega ($\omega$)-alkoxypolyalkoxides having the general formula

$$R^4O(CH_2CH(R^2)O)_nCH_2CH(R^2)O)_yM(OCH(R^2)CH_2(OCH(R^2)CH_2)_mOR^3)_{2-y}\bullet(R^1OH)_x \qquad (5)$$

wherein M is selected from magnesium, zinc and mixtures thereof, $R^3$ and $R^4$ are independently selected from $C_1$ to $C_{18}$ hydrocarbyl groups, $R^1$ is selected from $C_1$ to $C_{18}$ hydrocarbyl groups and a $R^3O(CH_2CH(R^2)O)_nCH_2CH(R^2)$ group, n and m are values from 1 to 20, $R^2$ is selected from hydrogen and methyl, y is a value from 0.01 to 1.0 and x is a value from zero to two.

The resulting carbonated products have carbon dioxide ($CO_2$) incorporated into only one side of the metal alkoxide resulting in a $CO_2/Mg$ ratio of about one, and possessing one of the following two structures:

$$(R^4O(CH_2CH(R^2O)_nCH_2CH(R^2)O)_yM(OC(O)OCH(R^2)CH_2(OCH(R^2)CH_2)_mOR^3)_{2-y}\bullet(R^1OH)_x \qquad (6)$$

and

$$(R^4O(CH_2CH(R^2)O)_nCH_2CH(R^2)OC(O)O)_yM(OCH(R^2)CH_2(OCH(R^2)CH_2)_mOR^3)_{2-y}\bullet(R^1OH)_x \qquad (7)$$

wherein M is selected from magnesium, zinc and mixtures thereof, n and m are values from 1 to 18, $R^2$ is hydrogen or methyl, $R^3$ and $R^4$ are independently selected from $C_1$ to $C_{18}$ hydrocarbyl groups, $R^1$ is selected from $C_1$ to $C_{18}$ hydrocarbyl groups and a $R^3O(CH_2CH(R^2)O)_nCH_2CH(R^2)$ group wherein $R^3$ is selected from $C_1$ to $C_{18}$ hydrocarbyl groups, y is a value from 0.01 to 1.0 and x is a value from between zero and two and a solvating amount of a solvent selected from aromatic hydrocarbons, halocarbons and mixtures thereof. Examples of these carbonated alkoxides are shown in Table 1.

Other magnesium and zinc alkoxides, which are a mixture of the types shown above with aliphatic or cycloaliphatic $C_1$ to $C_{18}$ metal alkoxides may also be half-carbonated to yield novel products of this invention. These metal alkoxides have the general formula:

$$(R^4O)_yM(OCH(R^2)CH_2(OCH(R^2)CH_2)_nOR^3)_{2-y}\bullet(R^1OH)_x \qquad (8)$$

wherein M is selected from magnesium, zinc and mixtures thereof, $R^3$ and $R^4$ are independently selected from $C_1$ to $C_{18}$ hydrocarbyl groups, $R^1$ is selected from $C_1$ to $C_{18}$ hydrocarbyl groups and $R^3O(CH_2CH(R^2)O)_nCH_2CH(R^2)$, n is value from 1 to 20, $R^2$ is selected from hydrogen and methyl, y is a value from 0.01 to 1.0 and x is a value from zero to two.

The resulting carbonated products possess one of the two following structures:

$$(R^4O)_yM(OC(O)OCH(R^2)CH_2(OCH(R^2)CH_2)_nOR^3)_{2-Y}\bullet(R^1OH)_x \qquad (9)$$

and

$$(R^4O(O)CO)_yM(OCH(R^2)CH_2(OCH(R^2)CH_2)_nOR^3)_{2-y}\bullet(R^1OH)_x \qquad (10)$$

wherein M is selected from magnesium, zinc, and mixtures thereof, n is a value from 1 to 20, y is a value from 0.01 to 1, $R^3$ and $R^4$ are independently selected from $C_1$ to $C_{18}$ hydrocarbyl groups, $R^2$ is selected from hydrogen and methyl, $R^1$ is selected from hydrocarbyl groups having one to eighteen carbon atoms and a $R^3O(CH_2CH(R^2)O)_nCH_2CH(R^2)$ group wherein $R^3$ is a $C_1$ to $C_{18}$ hydrocarbyl group, $R^2$ is selected from hydrogen and methyl, x is a value from zero to two and a solvating amount of a solvent selected from aromatic hydrocarbons, halocarbons and mixtures thereof. Examples of these carbonated metal alkoxides are shown in Table 2.

Aluminum and zinc alkoxides can be added to these carbonated compositions and to the other carbonated compositions of this invention to help solubilize the components of the compositions. According to another aspect this invention provides a composition suitable for use in the above described process comprising a mixture of a compound defined by (A) with an other compound defined by (B), wherein (A) is a substituted metal alkoxide having the formula

$$X_yM^a(OR)_{a-y}.(R^1OH)_x$$

EP 0 388 448 B1

(I) OR is a group selected from 2-alkoxyalkoxy- and $\omega$-alkoxypolyalkoxy- groups of the formula

$$[-OCH(R^2)CH_2(OCH(R^2)CH_2)_nOR^3]$$

wherein $R^2$ is selected from H and $-CH_3$ and $R^3$ is selected from alkyl groups of 1 to 18 carbon atoms, cycloalkyl groups of 3 to 18 carbon atoms and aryl, arylalkyl and alkylaryl groups of 6 to 18 carbon atoms and n is a value of 1 to 20 and wherein -OR is optionally carbonated;

(II) X is a group selected from

(a) 2-dialkylaminoalkoxy- and $\omega$-dialkylaminopolyalkoxy groups of the formula

$$[-OCH(R^{2'})CH_2(OCH(R^{2'})CH_2)_mNR^{3'}{}_2]$$

wherein $R^{2'}$ is selected from hydrogen and methyl, $R^{3'}$ is selected from alkyl groups of 1 to 18 atoms, cycloalkyl groups of 3 to 18 carbon atoms and aryl, arylalkyl and alkylaryl groups of 6 to 18 carbon atoms and m is a value of 1 to 20;

(b) a halogen selected from chlorine and bromine;

(c) an organic group $-R^4$ wherein $R^4$ is selected from the group consisting of alkyl groups containing 1 to 18 carbon atoms, cycloalkyl groups containing 3 to 18 carbon atoms and aryl, arylalkyl and alkylaryl groups containing 6 to 18 carbon atoms;

(d) an acyloxy group of the formula $[-O(O)CR^4]$ wherein $R^4$ has the hereintobefore ascribed meaning;

(e) alkoxypolyalkoxy- groups of the formula

$$[-OCH(R^{2'})CH_2(OCH(R^{2'})CH_2)_mOR^{3'}]$$

wherein $R^{2'}$, $R^{3'}$ and m have the hereintobefore ascribed meanings;

(f) an alkoxy group $-OR^4$ wherein $R^4$ has the hereintobefore ascribed meanings;

(III) M is a metal selected from groups IIa and IIb of the Periodic Table and aluminium and mixtures thereof;

(IV) $R^1OH$ is a compound in which $R^1O$ is a group selected from

(a) alkoxy groups of the formula $R^{4'}O$ wherein $R^{4'}$ has the meanings hereintobefore ascribed for $R^4$;

(b) 2-alkoxyalkoxy- and $\omega$-alkoxypolyalkoxy- groups of the formula

$$[-OCH(R^{2''})CH_2(OCH(R^{2''})CH_2)_oOR^{3''}]$$

wherein $R^{2''}$, $R^{3''}$ and o have the meanings hereintobefore ascribed for $R^2$, $R^3$ and o;

(c) 2-dialkylaminoalkoxy- and $\omega$-dialkylaminopolyakoxy groups of the formula

$$[-OCH(R^{2''})CH_2(OCH(R^{2''})CH_2)_nNR^{3''}{}_2]$$

(V) a is the valence of the metal M;

(VI) y has a value between zero and one; and

(VII) x has a value of zero to two; and

(B) is a carbonated substituted metal alkoxide having the formula

$$X_yM^a(OR)_{a-y}.(R^1OH)_x$$

in which either $X_y$ or -OR is carbonated wherein $R^1OH$, M, a, y and x have the hereintobefore ascribed meanings; and

(I) OR is a group selected from 2-alkoxyalkoxy- and $\omega$-alkoxypolyalkoxy- groups of the formula

$$[-OCH(R^2)CH_2(OCH(R^2)CH_2)_nOR^3]$$

wherein $R^2$ is selected from H and $-CH_3$ and $R^3$ is selected from alkyl groups of 1 to 18 carbon atoms, cycloalkyl groups of 3 to 18 carbon atoms and aryl, arylalkyl and alkyl-aryl groups of 6 to 18 carbon atoms and n is a value of 1 to 20; and

(II) X is a group selected from

(a) 2-dialkylaminoalkoxy- and $\omega$-dialkylaminopolyalkoxy groups of the formula

7

$[-OCH(R^{2'})CH_2(OCH(R^{2'})CH_2)_mNR^{3'}_2]$

wherein $R^{2'}$ is selected from hydrogen and methyl, $R^{3'}$ is selected from alkyl groups of 1 to 18 carbon atoms, cycloalkyl groups of 3 to 18 carbon atoms and aryl, arylalkyl and alkylaryl groups of 6 to 18 carbon atoms and m is a value of 1 to 20;

(b) an -alkoxypolyalkoxy- group of the formula

$[-OCH(R^{2'})CH_2(OCH(R^{2'})CH_2)_mOR^{3'}]$

wherein $R^{2'}$, $R^{3'}$ and m have the hereintobefore ascribed meanings; and,

(c) an alkoxy group $-OR^4$ wherein $R^4$ has the hereintobefore ascribed meaning.

The products of the invention may be prepared via a number of techniques, known to the art, such as by reacting a suspension of magnesium metal, or magnesium amide or $C_1$-$C_3$ magnesium dialkoxides in a hydrocarbon or halocarbon solvent, or by reacting a solution of a dialkylmagnesium or a dialkylzinc compound in a hydrocarbon solvent, with a 2-alkoxy-substituted alkanol ($ROCH_2CHR'OH$) wherein R is a $C_1$-$C_{12}$ hydrocarbyl and R' is hydrogen or methyl; or a member of the group of $\omega$-alkoxy-poly(alkoxy)-alkanols, of the formula $RO(CH_2CH(R')O)_nCH_2CH(R')OH$ wherein R is a $C_1$-$C_{18}$ hydrocarbyl group, R' is a methyl group or hydrogen, and n is from 1 to about 100, and carbonating the products thereof.

The 2-alkoxyalkanols ($ROCH_2CHR'OH$) where R is hydrogen, also have the trade name Cellosolves (Union Carbide) and are exemplified by the substances Methyl Cellosolve™ ($CH_3OCH_2CH_2OH$), Butyl Cellosolve™ ($C_4H_9OCH_2CH_2OH$) and Hexyl Cellosolve ($C_6H_{13}OCH_2CH_2OH$).

The $\omega$-alkoxypoly(alkoxy)alkanols, of the formula ($RO(CH_2CH(R)O)_nCH_2CH(R')OH$), where n is one and R' is hydrogen, also have the trade name Carbitol and are exemplified by the substances Methyl Carbitol, Butyl Carbitol and Hexyl Carbitol. Higher n numbers, e.g. where n is 2, give Methoxy, Ethoxy, and Butoxytriglycol, and where n is 6 or more, Methoxy Polyethylene Glycols (trademark Carbowax MPEG, Union Carbide).

These can be used in mixtures thereof with each other or with $C_1$ to $C_{12}$ alcohols selected from the group of (a) aliphatic 2-alkyl-substituted $C_4$-$C_{12}$ primary monohydric alcohols; or (b) aliphatic $C_3$-$C_{12}$ secondary or tertiary alcohols; or (c) aliphatic $C_1$-$C_{12}$ primary linear unsubstituted alcohols; and removing hydrogen or ammonia which forms during the reaction.

The starting magnesium or zinc alkoxide may also be prepared by reacting a solid magnesium or zinc dialkoxide of the formula $Mg(OR)_2$ or $Zn(OR)_2$ in which R is a $C_1$-$C_{12}$ hydrocarbyl group, with at least two molar equivalents of a 2-alkoxy-substituted alkanol, $ROCH_2CHR'OH$, or an $\omega$-alkoxypoly(alkoxy)alkanol, RO-$(CH_2CH(R')O)_nCH_2CH(R')OH$ wherein R is a $C_1$-$C_{12}$ hydrocarbyl group and R' is hydrogen or a methyl group, and n is from 0 to about 100, isolating the resultant mobile liquid product, and dissolving same in a hydrocarbon or chlorinated hydrocarbon or halocarbon solvent of choice, or by reacting a solid magnesium dialkoxide with just one molar equivalent of a 2-alkoxyalkanol or $\omega$-alkoxypoly(alkoxy)alkanol in a hydrocarbon or halocarbon solvent, or by reacting dialkylmagnesium or dialkylzinc compounds of the formula $MgR_2$ or $ZnR_2$ in which P is a $C_1$-$C_{12}$ hydrocarbyl group, with at least two molar equivalents of a 2-alkoxy-substituted alkanol, $ROCH_2CHR'OH$, or $\omega$-alkoxypoly(alkoxy)alkanol or mixtures of such alkanols, in which R is a $C_1$-$C_{12}$ hydrocarbyl group and R' is hydrogen or a methyl group, some typical examples of which are as described in US-A-4,634,786, incorporated herein by reference, and then carbonating the product thereof.

After carbonation is complete, as evidenced by no further evolution of heat, the resulting liquid products and solutions thereof of the magnesium or zinc alkoxide carbonates are unexpectedly more fluid (less viscous) than the original magnesium or zinc alkoxide products and solutions before carbonation, especially in the case of the higher homologs where n is greater than one; this is evident even after complete removal of solvents. For example, a sample of magnesium butoxytriglycolate (n = 2) in heptane (before carbonation) was stripped of solvent and found to be so extremely viscous that the product would not flow. Even after addition of 20 weight percent of hexane, the product was still not fluid enough to pour. On the other hand, another sample of the same magnesium butoxytriglycolate, after carbonation and stripping, yielded a completely fluid, low viscosity liquid residue, which was readily pourable and which formed fluid pourable solutions in hexane at all concentrations. (Similar results are evident when zinc is used in place of magnesium.)

Comparison of this thinning effect on carbonation of the magnesium alkoxides of this invention with the result obtained on carbonation of other hydrocarbon-soluble magnesium alkoxide types described in US-A-4,634,786 (magnesium 2-alkyl-substituted alkoxides) and hydrocarbon soluble magnesium/aluminum alkoxide complexes described in US-A-4,246,383, US-A-4,426,316, and US-A-4,244,838 is striking. Treatment of a 0.6M solution of magnesium 2-methylpentyloxide solution in heptane with carbon dioxide, resulted in a solid gel while a much lower concentration of the same compound gave a precipitate rather than a solution on carbonation. In another case, a sample of a 1:1 complex of magnesium and aluminum n-butoxides dissolved in heptane/hexane was carbonated resulting in gelation of the solution. Neither gelation nor precipitation leads to a useful product for the purpose of evenly treating cellulosic materials. Alcoholic cosolvents which might aid in dissolving these products are not desirable for the purpose of deacidifying paper and books, as was mentioned earlier.

The carbonated magnesium or zinc alkoxides of this invention possess the property of essentially complete miscibility in a variety of normally liquid hydrocarbon solvents such as, for example, pentane, hexane, heptane, benzene, toluene, and cyclohexane as well as gaseous hydrocarbons such as ethane, propane, propylene and butanes. In addition, said carbonated magnesium or zinc alkoxides are soluble in normally liquid halocarbons, such as 1,1,2-trichlorotrifluoroethane, symmetrical tetrachlorodifluoroethane, perchlorethylene, chloroform and methylchloroform and mixtures thereof. Other gaseous halocarbons contemplated as solvents are chlorotrifluoromethane, chlorodifluoroethane and 1,2-dichlorotetrafluoroethane.

Another property inherent in the carbonated magnesium or zinc alkoxides of this invention is the great expandability of their volumes either alone or in solution on treatment with certain gases such as, for example, the hydrocarbon gases ethane, propane, and butane, or the chlorofluorocarbon or fluorocarbon gases such as fluoroform, hexafluoroethane, tetrafluoroethane, perfluoropropane, chlorotrifluoroethane, chlorodifluoroethane and 1,2-dichlorotetrafluoroethane and even gases such as carbon dioxide. This property of solution expandability was found to be useful in the treatment of cellulosic materials such as paper and books. Although solution expandability on gasification is not a new concept, it has been noted that such solution expansion must be controlled by judicious pressure regulation so as not to exceed the solubility of the magnesium or zinc compound in the combination of gaseous and liquid solvents. Beyond a certain pressure precipitation of the dissolved magnesium or zinc compounds occurs, leading to a non-homogeneous deposition of a deacidifying agent on the books. For example, ethane gas reproducibly gave expanded solutions of the magnesium or zinc compounds of this invention to at least twice their original volume under pressures of 3.448 MPa (500 psi) or less without thickening or precipitation of the solutions. In addition, the original volume of ungasified solution could be reversibly generated by venting of the dissolved ethane gas. It is important to note that precipitation of the magnesium compounds of this invention can be made to occur using ethane by raising the pressure above about 3.448 MPa (500 psi) (pressure depends upon the concentration of magnesium in the original solution and its solvent composition), but is undesirable for the purpose of treatment of cellulosic materials, leading to streaky, sticky finishes.

Similarly, carbon dioxide gas expands perchloroethylene solutions of the carbonated magnesium or zinc alkoxides to over twice their original volume at pressures not much above 4.482 MPa (650 psi).

Thus, this property of expandability of hydrocarbon and halocarbon solutions of the magnesium or zinc compounds of this invention was found to be applicable to the treatment of cellulosic materials such as paper and books, by the simple expedient of suspending the cellulosic materials just above the level of the liquid hydrocarbon solutions of the magnesium or zinc compounds, pressurizing the hydrocarbon or halocarbon solutions with ethane or carbon dioxide gas to an internal pressure of about 3.448 MPa (500 psi) [4.482 MPa (650 psi) for $CO_2$], so as to cause the original solution volume to expand sufficiently (at least double) to cover the said cellulosic material, and, after a sufficiently long treatment time, to vent the dissolved ethane or carbon dioxide gas, thus reducing the volume of the hydrocarbon (or halocarbon) solution to its original volume and below the level of the treated cellulosic material. On removal from the pressure chamber, the treated cellulosic material was found to be dry and possessed a satiny smooth finish. Any number of consecutive treatments of the cellulosic material for varying lengths of time as described above can be carried out so as to impart the desired protective finish to the cellulosic material simply by alternatively expanding and contracting the hydrocarbon or halocarbon solution. Thus, for example, although single pieces of paper receive a deacidifying finish with as little as one such expansion/contraction cycle for as short a contact time as 15 seconds, multiple pieces of paper such as book pages may require several such cycles with longer contact time periods.

Generally, a pressure range for the carbonated magnesium alkoxide solution "expanding" gas should be from about 689.5 kPa (100 psi) to about 6.895 MPa (1000 psi). Supercritical gas pressure or temperatures are not required. In addition, elevated temperatures above ambient are not required during the expansion/contraction phases of the treatment of cellulosic materials.

It is also possible to use the hydrocarbon or halocarbon solutions of the carbonated zinc and magnesium alkoxides directly as deacidifying media, that is, without gaseous expansion. Thus, an approximately 8 volume/volume % (v/v%) solution of magnesium butoxytriglycolate (MBTG) in Freon TF (1,1,2-trichlorotrifluoroethane) was used to treat pages taken from a book, under various conditions, and found to evenly penetrate the pages, leaving about a 2% residual buffer of magnesium, calculated as $MgCO_3$. Strengthening of the pages was shown by an increase in the number of folds required to break a page in half (parallel to the binding) in the treated vs. the untreated samples, of about 400%. Other solvents may also be used, as mentioned above.

The concentration of the metal ion in the deacidifying treatment medium is in the range of 0.01 to 1.0 molar. More preferably, the range of metal ion concentration will lie in the range of 0.02 to 0.5 molar and, most preferably, 0.05 to 0.25 molar. Sufficient metal ion, calculated as $MgCO_3$, is generally desirable to provide about 2% $MgCO_3$ as both deacidifying agent and residual buffer, although this value may not be as critical where additional strengthening of the cellulosic material is being provided as by the products of this invention.

Naturally, the effects of time of treatment (treatment cycle) and number of treatment cycles is an important consideration and will also affect the metal ion concentration being deposited. Thus, in certain single page treatments, although about 2-5 minutes sufficed to give pages containing 2% of $MgCO_3$ - (equivalent), a 10 minute treatment time gave pages with the most even distribution of $MgCO_3$.

Another factor controlling metal ion deposition which needs to be empirically determined in each case is the type of cellulosic material being treated, which material can vary widely in composition.

As mentioned earlier for the gaseous expansion process, temperature and pressure will also play a role in metal ion deposition, and it is expected that higher values of both will promote (increase) metal ion concentrations in the treated products.

For such treatments, generally short cycles are preferable, of the the order of an hour or so or less, depending on the number and thickness of the items being treated, and on the porosity of the cellulosic material the items are made from. Thus, a 6.7 cm by 6.7 cm by 2.5 cm (3 inch by 3 inch by 1 inch) thick book containing porous paper was uniformly treated in 10 minutes, with a page taken from the center of the book showing the presence of about 1.5% $MgCO_3$.

It is believed that the magnesium and zinc compounds of this invention possess a unique chemical structure which can readily complex (associate) with the many hydroxyl groupings in cellulosic materials, thus binding the magnesium or zinc compounds tightly to the cellulosic materials and providing the latter with an additional (to deacidification) protective film or coating. As the number of ethoxy or propoxy units in the magnesium or zinc compounds increases, it has been found that the film or coating provided will also impart a strengthening property to the cellulosic material.

Thus, for example, the number of folds before breakage of pages taken from an old book which had been previously treated with the following magnesium alkoxide carbonates in a Freon TF Solution increased as shown:

| Magnesium Alkoxide (as the Carbonate) | No. of folds to break |
| --- | --- |
| Magnesium Butoxytriglycolate | 29 |
| Magnesium Butoxytriglycolate/Methoxypolyethoxide[a] | 35 |
| Magnesium Bis-Methoxypolyethoxide | 59 |
| None | 5 |
| Magnesium Methoxide | 4 |

(a) number of ethoxy units is an average of 7.4.

In contrast to previously utilized carbonated magnesium alkoxides (such as methoxymagnesium methyl carbonate) treatments where no strengthening effect is noted, not only do the magnesium salts of this invention adhere to the cellulosic structure of the paper, but the by-products of the deacidification process, the $\omega$-alkoxy(poly)ethoxy and $\omega$-alkoxy(poly)propoxy alcohols themselves, also do so. Thus, there is no massive venting of volatile alcohols, such as methanol, from the pages of a book during the continuing deacidification processes that may occur on library bookshelves in a book with the passage of time.

Metallic alkoxides of the type shown above for magnesium and zinc, but without carbonation, can also be used in such treatments, either alone, or in admixture with the carbonated magnesium and zinc alkoxides. Thus, for example, aluminum alkoxyalkoxides and $\omega$-alkoxypolyalkoxides can be prepared in pure liquid form and used alone or in admixture with the carbonated magnesium and zinc alkoxyalkoxides and alkoxypolyalkoxides described above. Typical aluminum alkoxides of this type are aluminum tris-hexylcarbitolate, aluminum tris-$\omega$-methoxypolyethoxide, ethoxyaluminum bis-$\omega$-methoxypolyethoxide, and the like.

10

Other partially alkoxylated alkylmetallic compounds may also be used in solution form to treat cellulosic materials. These alkylmetallic alkoxides have the generic formula:

$$(R^1)_yM^a(OCH(R^2)CH_2 \{ OCH(R^2)CH_2 \}_n OR^3)_{a-y}$$

wherein M is Mg, Zn, and Al and mixtures thereof, $R^1$ and $R^3$ are $C_1$ to $C_{18}$ hydrocarbyl, $R^2$ is hydrogen or methyl, n is a value from 1 to 20, y is a value between 0.01 and 1.0, and a is the valence of the metal.

Examples of these compounds are shown in Table III, below:

TABLE III

| $M^a$ | $R^1$ | $R^2$ | $R^3$ | n | y |
|---|---|---|---|---|---|
| Zn | $C_2H_5$ | H | $CH_3$ | 6.4 | 1.0 |
| Al | $i\text{-}C_4H_9$ | H | $CH_3$ | 6.4 | 1.0 |
| Mg | $\underline{n,s}\text{-}C_4H_9$ | H | $CH_3$ | 6.4 | 1.0 |
| Mg/Al = 2 | $\overline{C_4H_9}$ | H | $CH_3$ | 6.4 | 1.0 |
| Zn/Al = 1.5 | $C_2H_5/iC_4H_9$ | H | $CH_3$ | 6.4 | 1.0 |

Thus, for example, a solution of ethylzinc-$\omega$-methoxypolyethoxide in a halocarbon solution was used to treat books and found to give a uniform distribution of zinc in a page taken from the center of a book at a level of 1.5-1.7% zinc oxide.

Other such typical alkylmetal alkoxides which may be used alone or in combination with each other in solution form to deacidify and strengthen cellulosic materials are butylmagnesium $\omega$-methoxypolyethoxide, isobutylaluminum bis-$\omega$-methoxypolyethoxide and ethylzinc butoxytriglycolate.

It should be noted that the aluminum compounds, generally should not be used alone since they have a strong propensity toward hydrolysis.

In some cases it has been found that combinations of two or more of such alkylmetal alkoxides possess improved properties. Thus, for example, addition of an equivalent amount of isobutylaluminum-bis-$\omega$-methoxypolyethoxide to butylmagnesium-$\omega$-methoxypolyethoxide pacifies the latter's normal reactivity with halocarbon compounds to such an extent that a solution of these two metal alkoxides can be prepared containing a high concentration of Freon TF.

In some cases, these alkylmetal alkoxides may act to improve the solubility of the carbonated magnesium and zinc alkoxides in certain solvents such as Freon TF. Thus, for example, addition of an equal amount of isobutylaluminum bis-$\omega$-methoxypolyethoxide to zinc bis-methoxypolyethoxide (carbonated) improves the latter's solubility in Freon TF. By contrast, a Freon TF solution of aluminum isopropoxide did not promote the solubility of magnesium ethoxide in the same solvent. Such blends of metal alkoxides give deacidifying media which are milder buffers than magnesium alkoxides alone.

Also contemplated are the halometalalkoxides, $X_yM^a(OR)_{a-y}\cdot(R'OH)_x$, wherein X is a halogen, selected from chlorine and bromine, M is a metal of Periodic Groups IIA, IIB, or aluminium OR is an alkoxy group wherein R is an alkyl, cycloalkyl, aryl, arylalkyl or alkylaryl, or an alkoxyalkoxy group, particularly an 2-alkoxyalkoxy- or $\omega$-alkoxypoly(alkoxy) group, a is the valence of the metal, y is a value from zero to 1 and x is 0 to 2.

Examples of such halometalalkoxides are chloromagnesium butoxyethoxide, chloromagnesium $\omega$-methoxypolyethoxide, chlorozinc $\omega$-methoxypolyethoxide, chloroaluminum bis-$\omega$-butoxytriglycolate, and mixtures thereof.

A novel procedure for preparing such halometal alkoxides has been developed which involves reaction of the desired alcohol and metal in the presence of a minor quantity of methanol, and an equivalent quantity of fluorochlorocarbon such as 1,1,2-trifluorotrichloroethane (Freon TF) in a hydrocarbon solvent. The halocarbon functions as a halogenating agent in the process:

$$M^a + ROH + 0.5F_2ClC\text{-}CFCl_2 \rightarrow ClM^a(OR)_{a-1} + 0.5F_2C = CFCl$$

The hydrocarbon solution is filtered and stripped, if desired, to give residual liquid products, which can be redissolved in various solvents, described above.

Also contemplated are acyloxymetal alkoxyalkoxides, $(R^4C(O)O)_yM(\text{-}OCH(R^2)CH_2\{OCH(R^2)CH_2\}_nOR^3)_{2-y}\cdot(R'OH)_x$ and alkoxypolyalkoxides in which $R^4$ is a $C_1$ to $C_{18}$ alkyl, aryl, cycloalkyl, arylalkyl, or alkylaryl group, M is a divalent metal from Groups IIA and IIB of the Periodic Table, R' is a $C_1$ to $C_{18}$ alkyl, aryl,

cycloalkyl, arylalkyl, or alkylaryl group, or is alkoxyalkoxy or alkoxypolyalkoxy derived from the alkoxide, where $R^2$ is methyl group or hydrogen and R' and are the same or different alkyl, aryl, cycloalkyl, arylalkyl and alkylaryl, $R^3$ is a $C_1$ to $C_{18}$ alkyl y is a value from 0 to about 1 and x is a value from 0 to 2.

Also contemplated are magnesium and zinc mono- and dialkylaminoethoxides and dialkylaminopolyalkoxides such as, e.g., magnesium or zinc bis-N-methylaminoethoxide and magnesium or zinc bis-N,N-dimethylaminoethoxide, and their higher homologs,

$$R^3{}_2N(CH_2CH(R^2)O)_nCH_2CH(R^2)OMOCH(R^2)CH_2(OCH(R^2)CH_2)_mNR^3{}_2 \cdot (R^1{}_2NN)_x$$

where n and m are greater than 1 and $R^2$ may be H or $CH_3$ and $R^1$, and $R^3$ may be the same or different $C_1$-$C_{18}$ hydrocarbyl groups.

The following examples further illustrate the invention.

## EXAMPLES

Example I - Preparation of Carbonated Magnesium n-Hexyloxyethoxide $C_6H_{13}OCH_2CH_2O$-Mg-OC(O)-$OCH_2CH_2O$-$C_6H_{13}$ ("A")

To a solution of 292.5 g (2.0 mole) of ethyleneglycol, monohexyl ether (n-hexylcellosolve) dissolved in 850 ml heptane was slowly added 1.02 liters of a solution of dibutylmagnesium in heptane (19.2 weight percent). After reaction was complete, the solution was filtered and carbon dioxide passed into it through a gas inlet tube from a one liter flask containing pieces of dry ice. The gas was passed through two drying columns containing calcium chloride before entering the gas inlet tube. The temperature of the solution in the flask rose from 31° to 42°C during the carbonation. After reaction was complete, as noted by a significant drop in the reaction temperature, the solution was stripped of solvent on a ROTO-VAC apparatus and the residual fluid, somewhat viscous mass, transferred to a pint bottle under inert gas (argon). The weight of recovered product was 307 g (87% recovery).

Another 0.11 mole preparation of magnesium n-hexyloxyethoxide using magnesium ethoxide in place of dibutylmagnesium was carried out and the solvents (ethanol and heptane) removed by vacuum stripping. The viscous residue was redissolved in hexane (to about 0.9M) and carbonated as described above. The temperature during carbonation rose from 24.3° to 39.8°C. After 1.25 hours of slow $CO_2$ feed, the temperature had returned to 23°C. The solution was vacuum stripped to give a viscous mobile liquid that readily dissolved in hexane. Only a trace (<0.01%) of ethanol remained. An infrared scan of the residue dissolved in hexane showed a strong absorbence at 6 $\mu$m (microns) indicative of a carbonyl group. Analysis of the solvent-stripped residue for contained $CO_3$ gave a value of 16.5%, corresponding to a $CO_3$/Mg ratio of 1.01.

Example II - Preparation of Carbonated Magnesium Hexyloxyethoxyethoxide $C_6H_{13}(OCH_2CH_2)_2$-OMg-OC-(O)O-$(CH_2CH_2O)_2$-$C_6H_{13}$ ("C")

To 71.5 ml of a 27.4 weight percent solution of dibutylmagnesium in heptane (0.1 mole) in 100 ml of hexane was slowly added 41 ml (0.2 mole) of n-hexylcarbitol (neat). A clear, viscous solution was obtained upon cooling to room temperature. Carbonation of the solution with carbon dioxide gas thinned out the solution considerably. The resulting product was stripped of solvent to give 35 grams of a fairly fluid, somewhat viscous clear yellow mobile (pourable) liquid (87% recovery).

Example III - Preparation of Carbonated Ethoxymagnesium $\omega$-Methoxypolyethoxide $C_2H_5OC(O)OMg$-$OCH_2CH_2(OCH_2CH_2)_{6.4}OCH_3$ ("D")

To 71.5 ml of a 27.4 weight percent solution of dibutylmagnesium in heptane (0.1 mole) contained in 100 ml of toluene was slowly added 30 ml (0.093 mole) of methoxypolyethyleneglycol $(CH_3O(CH_2CH_2O)_{7.4}H$, Union Carbide Corp. MPEG 350). A clear, slightly hazy solution of the alkyl magnesium alkoxide resulted.

Two 10 ml aliquots of the solution (approx. 0.004 mole each) were taken and treated as follows:

(a) Carbonated with $CO_2$ - a clear very fluid solution was obtained on carbonation.

(b) Alkoxylated with ethanol. Sufficient ethanol (about 0.2 ml) was added to the aliquot to dissipate the red color generated by a small amount of 2,2'-biquinoline indicator. A thick, extremely viscous clear solution resulted, which on subsequent carbonation thinned out almost immediately to give a pale yellow clear, non-viscous solution.

Treatment of the remainder of the main alkyl-magnesium alkoxide solution as in (b) above, followed by solvent stripping of the carbonated magnesium alkoxide solution yielded 34 grams of a pale yellow, slightly hazy, mobile, somewhat viscous but pourable, liquid residue, which dissolved readily in toluene, but not in hexane. To 8.3 grams of carbonated ethoxy magnesium-ω-methoxypolyethoxide was added 45 ml of Freon TF to give a 2 phase mixture. Addition of 0.8 grams of butoxytriglycol gave an almost clear solution, while a further addition of 0.8 g of butoxytriglycol gave a clear solution. Thus, as little as 20-50 mole % (10-25 wt %) of butoxytriglycol will promote solubility of ethoxy magnesium-ω-methoxypolyethoxide in Freon TF.

In the formula for "D" above, carbonation could as well have been shown to take place on the methoxy-ω-polyethoxide side of the molecule.

Example IV - Preparation of Carbonated Magnesium Butoxytriglycolate $C_4H_9O(CH_2CH_2O)_2CH_2CH_2OMgOC(O)OCH_2CH_2O(CH_2CH_2)_2OC_4H_9$ ("B")

(a) To 25 mls (0.031 mole) of 1M dibutylmagnesium in hexane solution plus an additional 50 ml of hexane was slowly added 12.8 g pure butoxytriglycol (Union Carbide Corp.). The solution remained clear and fluid throughout. After approximately 0.5 hours, carbon dioxide was passed through the solution to form the alkoxide carbonate. The final solution was clear and fluid at about 0.35M Mg and was solvent stripped on the ROTO-VAC apparatus for 1 hour at 70°C. The concentrated solution was viscous, but fluid (mobile). An infrared scan of a hexane solution of the product showed a new band at 6.0 $\mu$m (microns).

(b) To 27.7 grams (1.14 g.at.) of magnesium powder in 850 ml of n-heptane activated with 0.2 g of iodine and heated to 90° was added 10 ml of a mixture of 362 g (1.75 moles) of butoxytriglycol and 24 g (0.52 mole) of ethanol. Reaction was slow, so an additional 15 g of magnesium metal in the form of chips was added and heating resumed. After a time, reaction began slowly and mixed alcohol feed was continued. After the reaction was completed, about half the mixture was filtered (slow filtration) to give a rather viscous, clear, yellow solution. The remainder of the unfiltered mixture was carbonated and the resulting mix became quite fluid and was easily filtered. After carbonation of the first half of the solution (thinning of solution), the solutions were combined and solvent-stripped to yield 368 grams of a viscous, but mobile, pourable product.

The carbonated solution of magnesium butoxytriglycolate in heptane prior to stripping was approximately 0.9 molar in magnesium concentration, but about 50 weight percent in carbonated product, showing the high concentrations of these magnesium compounds attainable. Some of the magnesium butoxytriglycolate from a previous run, which had not been carbonated, was also solvent stripped. The resulting product was extremely viscous (not mobile) even with the addition of 20% by weight of hexane, thus demonstrating the unexpected decrease in viscosity of these magnesium alkoxide solutions on carbonation.

The carbonated product was analyzed for carbonate ($CO_3$) content and found to contain 16.21 weight percent which corresponded to a $CO_3$/Mg ratio of 1.02. Infrared analysis showed a strong absorption in the 6.0-6.1 $\mu$m (micron) frequency region.

Example V - Preparation of Carbonated Magnesium Butoxy 2-Propoxide $C_4H_9OCH_2CH(CH_3)OMgOC(O)OCH(CH_3)CH_2OC_4H_9$

0.025 Mole (18 ml) of 27.4 weight percent DBM in heptane and 25 ml of hexane were placed in a flask and 7.5 ml (0.05 moles) of butoxy-2-propanol Propasol B (Union Carbide Propasol B, MW = 132.2, d = 0.8843) added dropwise with stirring. The solution remained hazy almost throughout the addition, then cleared up near the end of the addition to give a clear colorless, somewhat viscous solution. The product was carbonated and the viscosity of the solution appeared to decrease as evidenced by the greater stirring speed of the magnetic stirring bar.

Example VI - Preparation of Carbonated Hexyloxyethoxymagnesium ω- Methoxypolyethoxide (MPEG 350) $C_6H_{13}OCH_2CH_2OC(O)OMgOCH_2CH_2(OCH_2CH_2)_{6.4}OCH_3$

To 0.25 mole (18 ml) of 27.4 weight percent dibutylmagnesium solution in heptane (DBM) and 25 ml of hexane, was slowly added 8 ml (0.025 mole) of MPEG 350 (Union Carbide Methoxypolyethylene glycol, MW = 335-365). Next 4.1 ml (0.025 mole) of hexylcellosolve was added to yield a milky solution which slowly separated into two clear, colorless, mobile liquid layers. Next, the following amounts of DBM and hexylcellosolve (HC) were added in consecutive fashion: 9 ml (0.0125 mole) DBM, 4.1 ml (0.025 mole) HC,

13

9 ml DBM, 4.1 ml HC, 4.5 ml DBM, 2.05 ml HC, 4.5 ml DBM, 2.05 ml HC. The total DBM added to this point was 0.0625 mole, total hexylcellosolve was 0.100 mole and MPEG 350, 0.025 mole (ratio HC/MPEG = 4.0). A clear, colorless solution was obtained at warmer than room temperatures, but a small amount of a second phase separated on cooling to room temperature. On carbonation, some decrease in viscosity was noted, but solvent evaporation occurred during carbonation to give a slightly viscous, approximately 1 molar solution which was crystal clear and colorless even at room temperature. In the formula above, carbonation could as well have been shown to take place on the methoxy $\omega$-polyethoxide side of the molecule.

Example VII - Preparation of Carbonated Hexyloxyethoxyethoxymagnesium $\omega$-Methoxypolyethoxide

$C_6H_{13}OCH_2CH_2OCH_2CH_2OC(O)OMgOCH_2CH_2(OCH_2CH_2)_{6.4}OCH_3$

To 0.025 mole (18 ml) of DBM (27.4 weight percent in heptane) and 25 ml hexane was slowly added 8.1 ml (0.040 mole) of diethylene gylcol monohexyl ether (Hexyl Carbitol™ UCC) followed by 3 2 ml (0.01 mole) of MPEG 350. A clear, pale yellow fluid solution was obtained which, on carbonation, became even more fluid (less viscous). In the formula above, carbonation could as well have been shown to take place on the methoxy $\omega$-polyethoxide side of the molecule.

Example VIII - Preparation of Carbonated Magnesium Propoxy 2-Propoxide

$C_3H_7OCH_2CH(CH_3)OMgOC(O)OCH(CH_3)CH_2OC_3H_7$

To 0.025 mole (18 ml) of DBM (27.4 weight percent) and 25 ml hexane was slowly added 6.6 ml (0.05 mole) of propoxy 2-propanol (UCC Propasol P). The solution remained hazy almost throughout the addition, then cleared up to give a colorless, slightly viscous, mobile solution. Carbonation decreased the viscosity of the solution.

Example IX - Preparation of Zinc Hexyloxyethoxyethoxide

$C_6H_{13}OCH_2CH_2OCH_2CH_2OZnOCH_2CH_2OCH_2CH_2OC_6H_{13}$

To 50 ml of a 14.4 weight per cent solution of diethylzinc in hexane (density = 0.708) was added 16.8 ml (15.7 g, 0.082 m) hexyl carbitol (Union Carbide). Ethane was evolved during the addition and the temperature of the reaction mixture reached 55-60°C. After addition was complete, the mixture was heated to reflux for 30 minutes and then solvent was stripped on a ROTO-VAC unit to 80°C under full vacuum (< 1 min.). A deep amber, clear, viscous, but quite mobile liquid was obtained. Carbonation of a solution of this product in hexane at room temperature did not form a carbonate as evidenced by $CO_2$ evolution on treatment with aqueous HCl.

A repeat of the above preparation using milder temperatures (<30°C) yielded a light yellow colored, less viscous product.

Example X - Preparation of Aluminum Hexyloxyethoxyethoxide $Al(OCH_2CH_2OCH_2CH_2OC_6H_{13})_3$

A volume of 27 ml (25 g, 0.1325 m) of hexyl carbitol (Union Carbide) was added slowly to a solution of 50 ml of 25 weight per cent triisobutylaluminum in hexane (density 0.70). Isobutane was evolved and the solution heated to 55-60°C. The reaction mixture was then refluxed for 30 minutes and stripped of solvent under vacuum in a ROTO-VAC unit. The residual product was a fluid, slightly viscous, light yellow colored oil (24.5 grams).

Example XI - Preparation of Carbonated Magnesium-bis-$\omega$-methoxypolyethoxide

$CH_3O(CH_2CH_2O)_{6.4}CH_2CH_2OMgOC(O)OCH_2CH_2(OCH_2CH_2)_{6.4}OCH_3$

To 50 ml of a 1.3 molar solution of dibutylmagnesium in heptane (0.065 m) and 70 ml of toluene containing a small amount of 2,2'-biquinoline indicator, was added, slowly, 42 ml (46 g) of methoxypolyethylene glycol (Union Carbide MPEG 350) with stirring and cooling to a somewhat hazy, light yellow solution. The product was then treated with dry carbon dioxide gas for approximately one hour to give a clear solution. The solution was stripped under vacuum to a yellow-green, somewhat hazy mobile, viscous liquid (41.5 g).

A solution of this product in Freon TF could be prepared by admixture of as little as 14 wt % (50 mole %) of butoxytriglycol.

### Example XII - Preparation of Carbonated Butoxytriglycoxymagnesium-ω-methoxypolyethoxide

$C_4H_9O(CH_2CH_2O)_2CH_2CH_2OC(O)OMgOCH_2CH_2(OCH_2CH_2)_{6.4}OCH_3$

(a) To 54 ml of a 1.3 molar solution of dibutylmagnesium in heptane and 50 ml of toluene was added 22.4 ml (24.5 g, 0.07 m) of methoxypolyethylene glycol (MPEG 350, Union Carbide) and 14.4 ml (14.4 g, 0.07 m) of butoxytriglycol (Union Carbide). The solution stayed clear and fluid throughout the addition. The solution was carbonated to give a clear, fluid light colored solution. After stripping under vacuum to remove solvent, a weight of 43.7 g was obtained, which readily dissolved in Freon TF.

In the above formula, carbonation could have been shown to take place on the ω-methoxypolyethoxide group.

### Example XIII - Preparation of Ethyl Zinc ω-methoxypolyethoxide (EZMPG)

$C_2H_5ZnOCH_2CH_2(OCH_2\text{-}CH_2O)_{6.4}OCH_3$

To 121 ml of a 14.4 weight percent solution of diethylzinc in hexane (0.10 mole) was added slowly, 31.9 ml (35 g, 0.10 mole) of methoxypolyethylene glycol (MPEG 350, Union Carbide), keeping the temperature below 30°C with cooling. After stirring for an additional 45 minutes, the milky solution (2 layers) was vacuum stripped to remove hexane (only one liquid layer after strip). A grayish hazy liquid (39 g) was obtained which was dissolved in toluene, the solution was allowed to settle, and the clear supernatant decanted and again stripped to remove toluene. 35 g of an almost clear, water white fluid product was obtained, which slowly gave off a gas (bubbled) on hydrolysis or exposure to air, but did not spontaneously ignite. To 33 grams of EZnMPG was added 100 ml of Freon TF to give a slightly hazy solution.

### Example XIV - Preparation of Isobutylaluminum-ω-methoxypolyethoxide

$C_4H_9Al(OCH_2CH_2(OCH_2CH_2)_{6.4}OCH_3)_2$

To 113 ml of a 25 weight per cent solution of triisobutylaluminum in hexane (0.10 mole) was slowly added 69.4 ml of methoxypolyethylene glycol (MPEG 350, Union Carbide). The solution became milky and eventually formed two layers. Addition of 75 ml of toluene gave a clear solution. The solution was then vacuum stripped to remove solvents. A fluid, colorless, slightly hazy liquid (68 g) was recovered, which readily dissolved in Freon TF to give a clear solution.

### Example XV - Preparation of Butylmagnesium ω-methoxypolyethoxide

$C_4H_9MgOCH_2CH_2(OCH_2CH_2)_{6.4}OCH_3$

To 113 ml of a 0.97 m solution of dibutylmagnesium in heptane and 50 ml of toluene was added, with cooling, 35 ml (0.11 m) of MPEG 350 (Union Carbide). The mixture was then stirred for 2 hours and allowed to settle overnight. Two liquid layers were obtained, the lower layer containing 92% of the magnesium content. The upper layer was separated and discarded. 20 ml of toluene was added and the lower layer was vacuum stripped to give a clear orange-colored viscous liquid (42.3 g), which was very reactive to air and moisture, but not pyroforic. It reacted vigorously with Freon TF. However, when mixed with an equivalent amount of Isobutylaluminum ω-methoxypolyethoxide, only a very slight, if any, reaction with Freon TF was noted, indicating passivation of the carbon-magnesium bond and good solubility in Freon TF with potential for book deacidification.

### Example XVI - Preparation of Carbonated Zinc bis-ω-methoxypolyethoxide

$CH_3O(CH_2\text{-}CH_2O)_{6.4}CH_2CH_2OZnOC(O)OCH_2(OCH_2CH_2)_{6.4}OCH_3$

To a volume of 139 ml (0.115 mol) of a 14.4 weight percent solution of diethylzinc (DEZ) in hexane and 75 ml toluene was slowly added 80.5 g (0.23 mol) of methoxypolyethylene glycol (Average Mol. Wt = 350) at 25-30°C. The mixture (2 layers) was stirred 45 minutes after the DEZ addition was complete. The product mixture was vacuum stripped to remove solvent, but became extremely viscous and would not flow. The product mass was redissolved in hot toluene (40-50°C) and carbonated. Heat was generated as carbonation proceeded. The product solution was again vacuum-stripped to give a much less viscous, pourable product.

An infrared spectrum of the neat product showed bands at 5.15 (ms), 5.40 (ms), 5.55 (ms), 5.75(w), and 6.25 (s) μm (microns), all indicative of carbonyl groupings. There were no absoprtions in the 2.75-3.25 μm (microns) region indicative of hydroxyl groups. The product was not soluble in Freon TF; however, addition of 5 volume per cent chloroform gave a clear solution containing 5 volume per cent product. Admixture of the neat product with an equal volume of isobutylaluminum bis-ω-methoxypolyethoxide followed by Freon TF addition gave a fluid upper layer containing 67 vol % of metal MPEG compounds in Freon TF, indicating a solubilizing assistance of the aluminum compound.

Example XVII - Preparation of Carbonated Methoxymagnesium ω-ethoxytriglycolate in Freon TF

0.131 moles (11.30 g) of $Mg(OCH_3)_2$, 85 ml of Freon TF and 0.131 moles (23.2 g, 23 ml) of of ethoxytriglycol (Union Carbide Corp.) were mixed together and stirred for 30 minutes. Carbon dioxide was sprayed into the thick mass, causing the mix to thin and to become almost clear. An additional 40 ml of Freon TF was added along with 13.5 ml (0.066 moles) of butoxytriglycol and the solution diluted to 650 ml with Freon TF to give a 0.2 molar solution of the title compound.

Comparison Freon TF Solubility Test - Magnesium Ethoxide/Aluminum Isopropoxide

One gram (5 mmoles) of aluminum ispropoxide was dissolved in 10 ml of Freon TF solvent and 0.7 grams (6 mmoles) of magnesium ethoxide added. After mixing in an ultra-sonic bath, followed by settling of suspended matter, 3 ml of the clear solution was analyzed for Mg and Al. Theory Al: 1.5 mmole, Found Al: 1.5 mmole. Theory Mg: 1.8 mmoles, Found Mg: 0.08 mmoles. Conclusion: Little effect of Aluminum alkoxide on solubility of Magnesium alkoxide in Freon TF, and therefore not a useful system for deacidification of books.

Basically, the gas expansion experiments were carried out in two types of apparatus. First, a small amount of the metal compound to be tested was dissolved in the desired solvent to an approximately 10% (by volume) level, and solubility tests run in a Jurgeson Gauge with the desired expander gas (see description below). If a reasonable expansion was evidenced on pressurization with the gas without precipitation of the compound, further testing was carried out on 6.7 cm by 6.7 cm (3 inch by 3 inch) sections of books (generally pre-dried before treatment) in a 3 liter steel pressure vessel.

The first type of test apparatus was a Jurgeson Gauge connected to a source of pressurized gas. The Jurgeson Gauge is essentially a steam boiler sight glass in which volume expansions of samples by test gas could be readily observed and measured. The sample, usually dissolved in a compatible solvent, is transferred under inert gas into the bottom of the gauge, its liquid level height measured (1,2,3 "bolts" on the front of the gauge) and the desired test gas pressured in slowly, measuring the volume expansion of the liquid samples as the pressure is increased incrementally. Generally, at some specific pressure and volume, the sample product becomes insoluble in the expanded solvent medium and precipitates. Then, as the pressure is slowly released from the system, the product redissolves. In this way, the solubility parameters for each test gas and sample solution combination can be measured and optimum book treatment conditions determined.

The three samples tested were given letter designations A, B, and C and were essentially free of solvent. Sample A was product from EXAMPLE I, Sample B was product from EXAMPLE IV (b), Sample C was product from EXAMPLE II, and Sample D was product from EXAMPLE III.

A test was run in the Jurgeson Gauge with a solution of Sample C in hexane (approximately 33 weight percent), pressurizing with $CO_2$ to about 6.206-6.895 MPa (900-1000 psi), less than a doubling of volume resulted before the inception of precipitation of product from solution. Exhaustion of gas with pressure reduction caused dissolution of the precipitated product. A 10 volume percent solution of Sample C in perchlorethylene, pressurized to 5.999 MPa (870 psi) with $CO_2$, doubled in volume. The equivalent zinc and aluminum salts in perchlorethylene also showed this doubling effect.

Sample A solubilized well in Freon TF (20 weight percent) and a test was run with it and $CO_2$ in the Jurgeson Gauge. As the pressure was increased to about 3.448 MPa (500 psi), a thick viscous mass was formed in the gauge, which reverted back to a fluid solution on release of pressure. On running the same test with hexane in place of Freon TF, reversible precipitation occurred, but no thickening.

Sample B solubilized well in perchlorethylene, and a 10 volume percent solution was expanded with $CO_2$ to about 4.827 MPa (700 psi) in the Jurgeson Gauge with an apparent doubling of the original solution volume before there was any indication of precipitation. In hexane, only about a 30% volume expansion occurred at about 4.137 MPa (600 psi) before precipitation was noted.

Both pre-dried and undried 6.7 cm by 6.7 cm by 2.5 cm (3 inch by 3 inch by 1 inch) sections of an old book were treated with a $CO_2$ expanded at 4.482 MPa (650 psi) 10 vol percent solution of Sample B in perchlorethylene (400 ml) in a 3 liter tubular pressure vessel for 10 minutes at room temperature. The books were positioned just above [0.64 to 1.27 cm (1/4 to 1/2 in.)] the level of the liquid prior to

pressurization with $CO_2$. The pressure was first increased to 3.448 MPa (500 psi) and the liquid drained off, then the pressure bled off to zero. The vessel was once again pressured to 6.895 MPa (1000 psi) with $CO_2$ for a few minutes and then the pressure released ($CO_2$ "rinse"). The undried book exhibited a white residue on the inside and outside of the covers and the pages close to the covers, with a "dusting" effect throughout. The pre-dried book showed no evidence of a white residue or any dusting. A page taken from the center of the pre-dried treated book was sprayed with a pH indicator solution and showed that an even distribution of deacidifying agent had been deposited thereon. ICP analysis for % Mg (as $MgCO_3$) of a page taken from the center of the book and cut into quarters gave the following results: Upper left quarter: 1.05; upper right quarter: 1.40; lower left quarter: 0.91; lower right quarter: 1.12. These results also indicated a fairly even deposition of deacidifying compound into the books. Cold water extraction (70 ml) of 1 gram of the pages taken from the center of the book (TAPPI T-509) gave a pH value of 8.3.

A rough comparison of the strength of the treated vs. untreated pages taken from the center of the book was made by determining the number of times the pages could be folded back and forth (fold axis parallel to binding) before breakage and separation. The untreated page showed only 3 folds to break, the treated, 21.

Tests were run in the Jurgeson Gauge using Compound A solubilized in hexane, but with ethane as the expanding gas.

With an 8.6% solution of A in hexane, a precipitate was noted at about 4.137 MPa (600 psi). This test was repeated enclosing a sheet of rolled up paper in the gauge, set above the initial liquid level in the gauge (before pressuring with ethane). After expansion of the liquid level onto the paper surface and precipitation of product, the paper strip showed striations on testing with pH paper indicating coalescence of the droplets of viscous product and uneven deposition onto the paper. The test was repeated at an ethane pressure below 3.448 MPa (500 psi) which did not cause precipitation of the product, but which was sufficient to expand the solution volume to cover part of the test paper strip. After release of pressure, a dry paper was obtained. The immersed section of paper showed an even distribution of product onto the strip (pH indicator) giving the paper a satiny, smooth feel.

Another sample of A was dissolved in hexane (6 vol percent) and used to treat an old pre-dried book in the 3 liter pressure vessel using ethane as the expander gas at 3.448 MPa (500 psi). The book was positioned above the liquid level before pressurization. Treatment time was approximately one minute. After drying, a treated page was cut into quarters and analyzed for % Mg (as $MgCO_3$) in each quarter. Results were as follows: Upper left quarter: 0.35; upper right quarter: 0.95; lower left quarter: 1.02; lower right quarter: 1.61. Even for only such a short treatment period, a significant deposition of magnesium had occurred in the book. Obviously, a longer treatment time is required for even deposition throughout the book.

The solution (approximately 5 vol/vol %) of EZMPG in Freon TF/PERC in EXAMPLE XIII was used to treat pre-dried 6.7 cm by 6.7 cm by 1.27 cm (3 inch by 3 inch by 0.5 inch) section of paperback novel by immersion in the circulating solution for a period of 20 minutes. After removal of the solution and drainage of any excess, the wet book was dried by stripping under vacuum (35-40°C) for a period of two hours. A pH indicator solution was sprayed onto a page of the book taken from the center and indicated an even distribution of deacidifying agent had been deposited. pH measurement by the TAPPI T-509 Cold Water Extraction test showed a pH of 8.0 (vs. 6.3 in an untreated book). Determination of evenness of deposition of deacidifier by ICP measurement of zinc (expressed as % ZnO) in a center page of the book cut into four equal squares gave the following results: Upper left corner: 1.43; upper right corner: 1.70; lower left corner: 1.48; lower right corner: 1.70. The untreated book showed no more than 0.005% ZnO in any square.

An 8 vol % solution of Compound B (EXAMPLE IV (b)) in Freon TF was prepared by adding 600 ml of solvent to 49.4 grams of B. The solution was used to treat books and pages from these books under a variety of conditions:

(a) Single page treatment: Triangular, free-standing forms were made up of three pages taken from a pre-dried book and stapled together. This form was placed in the treatment unit, treated for various times with the above TF solution of B, then dried by vacuum stripping. The dry pages were separated, and tested for (a) pH by Cold Water Extraction (TAPPI T-509), percent magnesium distribution in the quartered page by ICP, and number of folds to break (strength). The following Table shows the results obtained:

| Treatment Time min. | pH | Folds to Break | % Mg (ICP) in quartered page (as $MgCO_3$) | | | |
|---|---|---|---|---|---|---|
| | | | UL | UR | LL | LR |
| 2 | 8.6 | 4 | 2.13 | 2.03 | 2.31 | 2.28 |
| 5 | 8.7 | 16 | 2.31 | 2.35 | 2.52 | 2.59 |
| 10 | 9.1 | 30 | 2.87 | 2.84 | 2.84 | 2.73 |
| UL = upper left; UR = upper right; LL = lower left; LR = lower right | | | | | | |

The results indicated that longer treatment times improved the strength of the page as well as the evenness of distribution and amount of deposited deacidifying agent in the page.

Further single page fold endurance tests were run comparing treated solution B in Freon TF with (a) Carbonated Butoxytriglycoxymagnesium-ω-methoxypolyethoxide (compound of EXAMPLE XII) in Freon TF (7.2 vol %), and (b) Carbonated Magnesium-bis-ω-methoxypolyethoxide (compound of EXAMPLE XI) in perchlorethylene (8.3 vol %).

Single 6.7 cm by 6.7 cm (3 inch by 3 inch) pages from an old book were stapled together as before and treated with the above solutions.

Results of the tests are as follows:

| Solution | No. of folds to break (Avg 3 tests) |
|---|---|
| B in TF | 29 |
| Comp'd of EX. XII in TF | 35 |
| Comp'd of EX. XI in PERC | 59 |
| Untreated | 7 |

These results indicate that longer poly(ethoxy) chains (7 ethoxy units in chain) impart greater strength to the pages than shorter chains (3 ethoxy units in the chain).

(b) Book Treatment: 3 inch by 3 inch by approximately 1 inch sections of an old book (both pre-dried and undried) were placed in the treatment vessel under inert gas (argon) and covered with the Freon TF solution of B used in (a) above. After 10 minutes, the treated solution was withdrawn, the books allowed to drain (15 minutes), and a vacuum applied [< 1 mm (133.22 Pa)] with exernal heating to about 35-40°C for 2 hours to dry the books. After returning the system to atmospheric pressure, the books were examined and tested as follows:

(i) Appearance: Undried book: white powdery patches on outside and inside of cover and throughout book. Pages have a powdery feel. Dried book: no white patches or powdery feel. Book appears unchanged from initial state.

(ii) pH (center page): undried book - 8.6; dried book - 8.7.

(iii) % Mg distribution (center page) as $MgCO_3$:

Undried book:    UL - 2.14; UR - 1.79; LL - 1.96; LR - 1.75.

Dried book:    UL - 1.44; UR - 1.44; LL - 1.51; LR - 1.54.

These results indicate that although deposition of deacidifier is greater in an undried book, as compared to a dried book, the deacidifier is not as evenly deposited, nor is it all deposited within the structure of the pages, i.e., some deacidifier is reacted with surface water and forms free (dusty) magnesium hydroxide, which is unacceptable. Pre-dried books were deacidified more evenly and completely and showed no obvious change in appearance before and after treatment.

TABLE I

$$(R^4O(CH_2CH(R^2)O)_nCH_2CH(R^2)O)_yM(OC(O)OCH(R^2)CH_2(OCH(R^3)CH_2)_mOR^3)_{2-y} \cdot (R^1OH)_x$$

and

$$(R^4O(CH_2CH(R^2)O)_nCH_2CH(R^2)OC(O)O)_yM(OCH(R^2)CH_2(OCH(R^2)CH_2)_mOR^3)_{2-y} \cdot (R^1OH)_x$$

| M | $R^4$ | $R^2$ | $R^3$ | $R^1$ | m | n | y | x |
|---|-------|-------|-------|-------|---|---|---|---|
| Mg | $C_6H_{13}$ | H | $C_6H_{13}$ | - | 0 | 0 | 1.0 | 0 |
| Mg | $C_6H_{13}$ | H | $C_6H_{13}$ | - | 1.0 | 1.0 | 1.0 | 0 |
| Mg | $C_4H_9$ | H | $C_4H_9$ | - | 2.0 | 2.0 | 1.0 | 0 |
| Mg | $C_4H_9$ | $CH_3$ | $C_4H_9$ | - | 0 | 0 | 1.0 | 0 |
| Mg | $C_6H_{13}$ | H | $CH_3$ | - | 6.4 | 0 | 1.6 | 0 |
| Mg | $C_6H_{13}$ | H | $CH_3$ | - | 6.4 | 1.0 | 1.6 | 0 |
| Mg | $C_3H_7$ | $CH_3$ | $C_3H_7$ | - | 0 | 0 | 1.0 | 0 |
| Zn | $C_6H_{13}$ | H | $C_6H_{13}$ | - | 1.0 | 1.0 | 1.0 | 0 |
| Mg | $CH_3$ | H | $CH_3$ | - | 6.4 | 6.4 | 1.0 | 0 |
| Mg | $C_4H_9$ | H | $CH_3$ | - | 2.0 | 6.4 | 1.0 | 0 |
| Zn | $CH_3$ | H | $CH_3$ | - | 6.4 | 6.4 | 1.0 | 0 |
| Mg | $CH_3$ | H | $CH_3$ | $C_4H_9(OCH_2CH_2)-CH_2CH_2-$ | 6.4 | 6.4 | 1.0 | 0.5 |

TABLE II

$$(R^4O)_yM(OC(O)OCH(R^2)CH_2(OCH(R^2)CH_2)_nOR^3)_{2-y} \cdot (R^1OH)_x$$

and

$$(R^4O(O)CO)_yM(OCH(R^2)CH_2(OCH(R^2)CH_2)_nOR^3)_{2-y} \cdot (R^1OH)_x$$

| $\underline{M}$ | $\underline{R^4}$ | $\underline{R^2}$ | $\underline{R^3}$ | $\underline{R^1}$ | $\underline{n}$ | $\underline{y}$ | $\underline{x}$ |
|---|---|---|---|---|---|---|---|
| Mg | $C_2H_5$ | H | $CH_3$ | - | 6.4 | 1.0 | - |
| Mg | $CH_3$ | H | $C_2H_5$ | $CH_3$ [1] | 2.0 | 1.0 | 1.0 |
|  |  |  |  | $C_4H_9(OCH_2CH_2)_2CH_2CH_2-$ |  |  | 0.5 |
| Mg | $CH_3$ | H | $C_4H_9$ | $CH_3$ | 2.0 | 1.0 | 1.0 |
| Mg | $CH_3$ | H | $CH_3$ | $CH_3$ [1] | 2.0 | 1.0 | 1.0 |
|  |  |  |  | $C_4H_9(OCH_2CH_2)_2CH_2CH_2-$ |  |  | 1.0 |
| Mg | $C_2H_5$ | H | $C_4H_9$ | $C_4H_9(OCH_2CH_2)_2$ $CH_2CH_2-$ | 2.0 | 1.0 | 0.5 |
| Mg | $C_2H_5$ | H | $CH_3$ | $C_4H_9(OCH_2CH_2)_2$ $CH_2CH_2-$ | 6.4 | 1.0 | 0.5 |
| Mg | $C_2H_5$ | H | $CH_3$ | $C_4H_9(OCH_2CH_2)_2$ $CH_2CH_2-$ | 6.4 | 2.0 | 0.27 |

(1)  $R^1$ is mixture of radicals

**Claims**

1.  A process for deacidifying cellulosic materials comprising contacting the cellulosic material with a solution in a hydrocarbon or halocarbon or mixtures thereof containing 0.01-1 mole/l of one or more substituted metal alkoxides having the formula

    $$X_yM^a(OR)_{a-y} \cdot (R^1OH)_x$$

    wherein:
    (I) OR is a group selected from 2-alkoxyalkoxy- and $\omega$-alkoxypolyalkoxy- groups of the formula

    $$[-OCH(R^2)CH_2(OCH(R^2)CH_2)_nOR^3]$$

    wherein $R^2$ is selected from H and $-CH_3$ and $R^3$ is selected from alkyl groups of 1 to 18 carbon atoms, cycloalkyl groups of 3 to 18 carbon atoms and aryl, arylalkyl and alkylaryl groups of 6 to 18 carbon atoms and n is a value of 1 to 20 and wherein -OR is optionally carbonated;

20

(II) X is a group selected from

(a) 2-dialkylaminoalkoxy- and $\omega$-dialkylaminopolyalkoxy groups of the formula

$$[-OCH(R^{2'})CH_2(OCH(R^{2'})CH_2)_mNR^{3'}{}_2]$$

wherein $R^{2'}$ is selected from hydrogen and methyl, $R^{3'}$ is selected from alkyl groups of 1 to 18 atoms, cycloalkyl groups of 3 to 18 carbon atoms and aryl, arylalkyl and alkylaryl groups of 6 to 18 carbon atoms and m is a value of 1 to 20;

(b) a halogen selected from chlorine and bromine;

(c) an organic group $-R^4$ wherein $R^4$ is selected from the group consisting of alkyl groups containing 1 to 18 carbon atoms, cycloalkyl groups containing 3 to 18 carbon atoms and aryl, arylalkyl and alkylaryl groups containing 6 to 18 carbon atoms;

(d) an acyloxy group of the formula $[-O(O)CR^4]$ wherein $R^4$ has the hereintobefore ascribed meaning;

(e) alkoxypolyalkoxy- groups of the formula

$$[-OCH(R^{2'})CH_2(OCH(R^{2'})CH_2)_mOR^{3'}]$$

wherein $R^{2'}$, $R^{3'}$ and m have the hereintobefore ascribed meanings;

(f) an alkoxy group $-OR^4$ wherein $R^4$ has the hereintobefore ascribed meanings;

(III) M is a metal selected from groups IIa and IIb of the Periodic Table and aluminium and mixtures thereof;

(IV) $R^1OH$ is a compound in which $R^1O$ is a group selected from

(a) alkoxy groups of the formula $R^{4'}O$ wherein $R^{4'}$ has the meanings hereintobefore ascribed for $R^4$;

(b) 2-alkoxyalkoxy- and $\omega$-alkoxypolyalkoxy-groups of the formula

$$[-OCH(R^{2''})CH_2(OCH(R^{2''})CH_2)_oOR^{3''}]$$

wherein $R^{2''}$, $R^{3''}$ and o have the meanings hereintobefore ascribed for $R^2$, $R^3$ and n;

(c) 2-dialkylaminoalkoxy- and $\omega$-dialkylamino-polyakoxy groups of the formula

$$[-OCH(R^{2''})CH_2(OCH(R^{2''})CH_2)_oNR^{3''}{}_2]$$

(V) a is the valence of the metal M;

(VI) y has a value between zero and one; and

(VII) x has a value of zero to two;

2. A process according to claim 1, **characterized in that** at least one of the compounds used is a compound of formula

$$X_yM^a(OR)_{a-y}.(R^1OH)_x$$

in which either $X_y$ or $-OR$ is carbonated wherein $R^1OH$, M, a, y and x have the hereintobefore ascribed meanings; and

(I) OR is a group selected from 2-alkoxyalkoxy- and $\omega$-alkoxypolyalkoxy groups of the formula

$$[-OCH(R^2)CH_2(OCH(R^2)CH_2)_nOR^3]$$

wherein $R^2$ is selected from H and $-CH_3$ and $R^3$ is selected from alkyl groups of 1 to 18 carbon atoms, cycloalkyl groups of 3 to 18 carbon atoms and aryl, arylalkyl and alkylaryl groups of 6 to 18 carbon atoms and n is a value of 1 to 20; and

(II) X is a group selected from

(a) 2-dialkylaminoalkoxy- and $\omega$-dialkylaminopolyalkoxy groups of the formula

$$[-OCH(R^{2'})CH_2(OCH(R^{2'})CH_2)_mNR^{3'}{}_2]$$

wherein $R^{2'}$ is selected from hydrogen and methyl, $R^{3'}$ is selected from alkyl groups of 1 to 18

21

carbon atoms, cycloalkyl groups of 3 to 18 carbon atoms and aryl, arylalkyl and alkylaryl groups of 6 to 18 carbon atoms and m is a value of 1 to 20;
(b) an -alkoxypolyalkoxy- group of the formula

$$[-OCH(R^{2'})CH_2(OCH(R^{2'})CH_2)_mOR^{3'}]$$

wherein $R^{2'}$, $R^{3'}$ and m have the hereintobefore ascribed meanings; and,
(c) an alkoxy group $-OR^4$ wherein $R^4$ has the hereintobefore ascribed meaning.

3. The process of claim 1 or 2 wherein the solution is a 0.02 to 0.5 molar solution of the substituted metal alkoxide.

4. The process of claim 3 wherein the solution is a 0.05 to 0.25 molar solution of the substituted metal alkoxide.

5. The process according to one of the preceding claims wherein said cellulosic materials are contacted with a solution in halocarbon or hydrocarbon or mixtures thereof of substituted metal alkoxides and their carbonated derivatives under a pressure of between 689.5 kPa and 6.895 MPa (100 and 1000 psi).

6. The process of one of the preceding claims wherein said substituted metal alkoxides are metal $\omega$-alkoxypolyalkoxides of the type $(R^3O(CH_2CH(R^{2'})O)_mCH_2CH(R^{2'})O)_yM^a(OCH(R^2)CH_2(OCH(R^2)CH_2)_nOR^3)_{a-y}.(R^1OH)_x$ wherein $R^2$ is a hydrogen or methyl group and $R^3$ and $R^{3'}$ are the same or different $C_1$ to $C_{18}$ alkyl groups, $R^1$ is a $C_1$ to $C_{18}$ alkyl, or $\omega$-alkoxypolyalkoxyalkyl group, a is the valence of the metal, n and m are values from 1 to 20, x is a value from zero to 2 and y is a value between 0.01 and 1.

7. The process of one of the preceding claims wherein said substituted metal alkoxides are alkylmetal 2-alkoxy-alkoxy-, and $\omega$-alkoxy-polyalkoxides $R^4_yM(OCH(R^2)CH_2(OCH(R^2)CH_2)_n-OR^3)_{a-y}$, where $R^2$ is selected from hydrogen and a methyl group and $R^4$ and $R^3$ are the same or different $C_1$ to $C_{18}$ alkyl groups, a is the valence of the metal, y is a value from 0.1 to 1.0, and n is a value from 1 to 20.

8. The process of one of the preceding claims wherein the substituted metal alkoxides are selected from the group of magnesium, zinc, aluminium alkoxides, and mixtures thereof.

9. The process of one of the preceding claims wherein said carbonated derivatives of said substituted metal alkoxides are carbonated magnesium and zinc alkoxides and possess a maximum of one carbonate group per metal atom.

10. The process of one of the preceding claims wherein said carbonated substituted metal alkoxides are alkoxymagnesium-, alkoxyzinc-2-alkoxyalkoxides and magnesium or zinc $\omega$-alkoxypolyalkoxides of the formula $(R^4O)_yM(OC(O)OCH(R^2)CH_2(OCH(R^2)CH_2)_nOR^3)_{2-y}.(R^1OH)_x$, wherein M is selected from magnesium and zinc, $R^2$ is hydrogen or a methyl group, $R^3$ and $R^4$ are the same or different $C_1$ to $C_{18}$ alkyl groups, $R^1$ is a $C_1$ to $C_{18}$ alkyl, 2-alkoxyalkyl or $\omega$-alkoxypolyalkoxyalkyl group, y is a value from 0.01 to 1, x is a value from zero to 2, and n is a value from 1 to 20.

11. The process according to one of the preceding claims wherein said carbonated derivatives of said metal alkoxides are selected from magnesium bis-butoxytriglycolate, magnesium bis-$\omega$-methoxy-polyethoxide, zinc bis-butoxytriglycolate, zinc bis-$\omega$-methoxypolyethoxide, magnesium bis-hexyl-carbitolate, zinc bis-hexyl-carbitolate, ethoxymagnesium $\omega$-methoxypoly-ethoxide, methoxymagnesiumethoxytri-glycolate, ethoxymagnesiumethoxy-triglycolate, methoxy-magnesiumbutoxytriglycolate and ethoxyzinc $\omega$-methoxy-polyethoxide, each member of the group complexed with from 0.2 to 0.5 molar equivalents of butoxytriglycol.

12. The process according to one of the preceding claims wherein said cellulosic materials are contacted with normally liquid halocarbon and hydrocarbon solutions of metal alkoxides and their carbonated derivatives at atmospheric pressure, followed by removal of said solutions from said cellulosic materials.

**13.** The process according to claim 6 wherein said alkylmetalalkoxyalkoxides and alkylmetal $\omega$-alkoxypolyalkoxides are selected from butylmagnesium $\omega$-methoxypolyethoxide, ethylzinc $\omega$-methoxypolyethoxide, butylaluminium bis-$\omega$-methoxypoly-ethoxide, and mixtures thereof.

**14.** The process according to claim 1 or 2 wherein the carbonated substituted metal alkoxides are selected from compounds having the formulae

$$(R^4O)_yM(OC(O)OCH(R^2)CH_2(OCH(R^2)CH_2)_nOR^3)_{2-y}.(R^1OH)_x$$

and

$$(R^4O(O)CO)_yM(OCH(R^2)CH_2(OCH(R^2)CH_2)_nOR^3)_{2-y}.(R^1OH)_x$$

wherein M is selected from Mg, Zn and mixtures of Mg and Zn, $R^3$ and $R^4$ are $C_1$-$C_{18}$ alkyl groups, $R^2$ is hydrogen or methyl, $R^1$ is selected from $C_1$-$C_{18}$ alkyl groups and a $R^{3''}O(CH_2CH(R^{2''})O)_oCH_2CH(R^{2''})$ group wherein $R^{2''}$ and $R^{3''}$ have the meanings given above for $R^2$ and $R^3$, y is a value from 0.01 to one, n and o are values from 1 to 20 and x is a value from zero to two.

**15.** The process according to claim 1 or 2 wherein the carbonated substituted metal alkoxides are selected from compounds having the formulae

$$(R^{3'}O(CH_2CH(R^{2'})O)_mCH_2CH(R^{2'})O)_yM(OC(O)OCH(R^2)CH_2(OCH(R^2)CH_2)_nOR^3)_{2-y}.(R^1OH)_x$$

and

$$(R^{3'}O(CH_2CH(R^{2'})O)_mCH_2CH(R^{2'})OC(O)O)_yM(OCH(R^2)CH_2(OCH(R^2)CH_2)_n OR^3)_{2-y}.(R^1OH)_x$$

wherein M is selected from Mg, Zn, and mixtures thereof, $R^2$ and $R^{2'}$ are hydrogen or methyl, $R^3$ and $R^{3'}$ are the same or different $C_1$-$C_{18}$ alkyl groups, $R^1$ is selected from $C_1$-$C_{18}$ alkyl groups and a $R^{3''}O$-$(CH_2CH(R^{2''})O)_oCH_2CH(R^{2''})$ group wherein $R^{3''}$ and $R^{2''}$ have the meanings indicated above for $R^2$ and $R^3$, y is a value from 0.01 to 1.0, n, m and o are values from 1 to 18 and x is a value from between zero and two.

**16.** The process according to claim 15 wherein M is magnesium, n is 2, m is 2, $R^{3'}$ is butyl, $R^3$ is butyl, $R^2$, $R^{2'}$ and $R^{2''}$ are hydrogen and x is zero.

**17.** The process according to claim 15 wherein M is magnesium, m is 6.4, n is 2, $R^{3'}$ is methyl, $R^3$ is butyl, $R^2$, $R^{2'}$ and $R^{2''}$ are hydrogen and x is zero.

**18.** The process according to claim 12 wherein the hydrocarbon solvent is selected from the group consisting of pentane, hexane, heptane, benzene, toluene, cyclohexane, ethane, propane, butane, propylene an mixtures thereof.

**19.** The process according to claim 12 wherein the halocarbon is selected from the group consisting of 1,1,1-trichlorotri-fluoroethane, symmetrical tetrachlorodifluoroethane, perchloroethylene, chloroform, methylchloroform, chlorotrifluoromethane, chlorodifluoroethane, 1,2-dichlorotetra-fluoroethane and mixtures thereof.

**20.** A composition suitable for use in the process of claim 1 to 19 comprising a mixture of a compound defined by (A) with an other compound defined by (B), wherein (A) is a substituted metal alkoxide having the formula

$$X_yM^a(OR)_{a-y}.(R^1OH)_x$$

(I) OR is a group selected from 2-alkoxyalkoxy- and $\omega$-alkoxypolyalkoxy- groups of the formula

$$[-OCH(R^2)CH_2(OCH(R^2)CH_2)_nOR^3]$$

wherein $R^2$ is selected from H and $-CH_3$ and $R^3$ is selected from alkyl groups of 1 to 18 carbon atoms, cycloalkyl groups of 3 to 18 carbon atoms and aryl, arylalkyl and alkylaryl groups of 6 to 18 carbon atoms and n is a value of 1 to 20 and wherein -OR is optionally carbonated;

(II) X is a group selected from

(a) 2-dialkylaminoalkoxy- and $\omega$-dialkylaminopolyalkoxy groups of the formula

$$[-OCH(R^{2'})CH_2(OCH(R^{2'})CH_2)_mNR^{3'}_2]$$

wherein $R^{2'}$ is selected from hydrogen and methyl, $R^{3'}$ is selected from alkyl groups of 1 to 18 atoms, cycloalkyl groups of 3 to 18 carbon atoms and aryl, arylalkyl and alkylaryl groups of 6 to 18 carbon atoms and m is a value of 1 to 20;

(b) a halogen selected from chlorine and bromine;

(c) an organic group $-R^4$ wherein $R^4$ is selected from the group consisting of alkyl groups containing 1 to 18 carbon atoms, cycloalkyl groups containing 3 to 18 carbon atoms and aryl, arylalkyl and alkylaryl groups containing 6 to 18 carbon atoms;

(d) an acyloxy group of the formula $[-O(O)CR^4]$ wherein $R^4$ has the hereintobefore ascribed meaning;

(e) alkoxypolyalkoxy- groups of the formula

$$(-OCH(R^{2'})CH_2(OCH(R^{2'})CH_2)_mOR^{3'}]$$

wherein $R^{2'}$, $R^{3'}$ and m have the hereintobefore ascribed meanings;

(f) an alkoxy group $-OR^4$ wherein $R^4$ has the hereintobefore ascribed meanings;

(III) M is a metal selected from groups IIa and IIb of the Periodic Table and aluminium and mixtures thereof;

(IV) $R^1OH$ is a compound in which $R^1O$ is a group selected from

(a) alkoxy groups of the formula $R^{4'}O$ wherein $R^{4'}$ has the meanings hereintobefore ascribed for $R^4$;

(b) 2-alkoxyalkoxy- and $\omega$-alkoxypolyalkoxy- groups of the formula

$$[-OCH(R^{2''})CH_2(OCH(R^{2''})CH_2)_oOR^{3''}]$$

wherein $R^{2''}$, $R^{3''}$ and o have the meanings hereintobefore ascribed for $R^2$, $R^3$ and n;

(c) 2-dialkylaminoalkoxy- and $\omega$-dialkylaminopolyakoxy groups of the formula

$$[-OCH(R^{2''})CH_2(OCH(R^{2''})CH_2)_oR^{3''}_2]$$

(V) a is the valence of the metal M;

(VI) y has a value between zero and one; and

(VII) x has a value of zero to two; and

(B) is a carbonated substituted metal alkoxide having the formula

$$X_yM^a(OR)_{a-y}.(R^1OH)_x$$

in which either $X_y$ or -OR is carbonated wherein $R^1OH$, M, a, y and x have the hereintobefore ascribed meanings; and

(I) OR is a group selected from 2-alkoxyalkoxy- and $\omega$-alkoxypolyalkoxy- groups of the formula

$$[-OCH(R^2)CH_2(OCH(R^2)CH_2)_nOR^3]$$

wherein $R^2$ is selected from H and $-CH_3$ and $R^3$ is selected from alkyl groups of 1 to 18 carbon atoms, cycloalkyl groups of 3 to 18 carbon atoms and aryl, arylalkyl and alkyl-aryl groups of 6 to 18 carbon atoms and n is a value of 1 to 20; and

(II) X is a group selected from

(a) 2-dialkylaminoalkoxy- and $\omega$-dialkylaminopolyalkoxy groups of the formula

$$[-OCH(R^{2'})CH_2(OCH(R^{2'})CH_2)_mNR^{3'}_2]$$

wherein $R^{2'}$ is selected from hydrogen and methyl, $R^{3'}$ is selected from alkyl groups of 1 to 18 carbon atoms, cycloalkyl groups of 3 to 18 carbon atoms and aryl, arylalkyl and alkylaryl groups of 6 to 18 carbon atoms and m is a value of 1 to 20;
(b) an -alkoxypolyalkoxy- group of the formula

$$[-OCH(R^{2'})CH_2(OCH(R^{2'})CH_2)_mOR^{3'}]$$

wherein $R^{2'}$, $R^{3'}$ and m have the hereintobefore ascribed meanings; and,
(c) an alkoxy group $-OR^4$ wherein $R^4$ has the hereintobefore ascribed meaning.

21. A compostion according to claim 20, comprising a mixture of compounds selected from compounds of the formula

$$(R^4O)_yM(OC(O)OCH(R^2)CH_2(OCH(R^2)CH_2)_nOR^3)_{2-y}.(R^1OH)_x$$

and

$$(R^4O(O)CO)_yM(OCH(R^2)CH_2(OCH(R^2)CH_2)_nOR^3)_{2-y}.(R^1OH)_x$$

wherein M is selected from Mg, Zn, mixtures of Mg and Zn, n is a value from 1 to 20, $R^3$ and $R^4$ are $C_1$-$C_{18}$ hydrocarbyl groups, $R^2$ is hydrogen or methyl and $R^1$ is the same or different $R^4$ and/or $R^{3''}O$-$(CH_2CH(R^{2''})O)_n$-$CH_2CH(R2'')$, y is a value from 0.01 to one and x is a value from zero to two.

22. The composition of claim 21 wherein M is magnesium, $R^3$ and $R^4$ are selected from methyl, ethyl and butyl, $R^2$ is hydrogen and $R^1$ is selected from methyl, ethyl, butyl and a $R^{3''}O(CH_2CHO)_2CH_2CH_2$- group in which $R^{3''}$ is selected from methyl, ethyl and butyl groups, n is two, y is one and x is zero to two.

23. The composition of claim 22 wherein n is 6.4, $R^3$ is methyl, $R^4$ is ethyl, $R^1$ is $C_4H_9O(CH_2CH_2O)$-$_2CH_2CH_2$- and y is one and x is 0.2 to 0.5.

24. The composition of claim 20 in which $R^2$ is ethyl, $R^4$ is selected from methyl and $C_4H_9O(CH_2CH_2O)$-$CH_2CH_2$- and x is 1.5.

25. The composition of claim 21 wherein M is magnesium, n is 2, $R^4$ is ethyl, $R^3$ is butyl, $R^2$ is hydrogen, $R^1$ is $C_4H_9O(CH_2CH_2O)_2CH_2CH_2$- and x is 0.5.

26. The composition of claim 21 wherein M is magnesium, n is 6.4, $R^3$ is methyl, $R^4$ is ethyl, $R^2$ is hydrogen, $R^1$ is $C_4H_9O(CH_2CH_2O)_2CH_2CH_2$-, and x is 0.25.

27. A composition according to claim 20 comprising a mixture of compounds selected from compounds of the formula

$$(R^{3'}O(CH_2CH(R^{2'})O)_mCH_2CH(R^{2'})O)_yM(OC(O)OCH(R^2)CH_2(OCH(R_2)CH_2)_n OR^3)_{2-y}.(R^1OH)_x$$

and

$$(R^{3'}O(CH_2CH(R^{2'})O)_mCH_2CH(R^{2'})OC(O)O)_yM(OCH(R^2)CH_2(OCH(R^2)CH_2)_n OR^3)_{2-y}.(R^1OH)_x$$

wherein M is selected from Mg, Zn, and mixtures thereof, n and m are values from 1 to 20, $R^2$ and $R^{2'}$ are hydrogen or methyl, $R^3$ and $R^{3'}$ are $C_1$-$C_{18}$ alkyl groups, $R^1$ is $R^{3''}O(CH_2CH_2O)_oCH_2CH_2$-, wherein $R^{3''}$ has the meaning previously ascribed to $R^3$ and o the meaning previously ascribed to n, y is a value from 0.01 to one and x is a value from between zero and two.

28. The composition of claim 27 wherein M is magnesium, m is 6.4, n is 2, $R^{3'}$ is methyl, $R^3$ is butyl, $R^2$ and $R^{2'}$ are hydrogen and x is zero.

**29.** The composition of claim 27 wherein M is magnesium, n is 2, m is 2, $R^{3'}$ is butyl, $R^3$ is butyl, $R^2$ and $R^{2'}$ are hydrogen and x is zero.

**30.** A process for preparing fluid, low viscosity carbonated metal 2-alkoxy- and $\omega$-alkoxypolyalkoxides comprising reacting in a solvent selected from liquid hydrocarbon solvents and liquid halocarbon solvents a reactant selected from substituted metal 2-alkoxyalkoxy- and metal $\omega$-alkoxypolyalkoxides of the formula

$$(R^4O)_yM^a(OCH(R^2)CH_2(OCH(R^2)CH_2)_nOR^3)_{a-y}.(R^1OH)_x$$

and

$$(R^{3'}O(CH_2CH(R^{2'})O)_mCH_2CH(R^{2'})O)_yM^a(OCH(R^2)CH(OCH(R_2)CH_2)_m OR^3)_{a-y}.(R^1OH)_x$$

wherein M is a metal selected from magnesium, zinc and mixtures of Mg and Zn, $R^2$ and $R^{2'}$ are selected from hydrogen and a methyl radical, $R^1$, $R^3$ and $R^{3'}$ are independently selected from alkyl groups containing 1 to 12 carbon atoms, y has a value from 0.01 to 1, x is a value from 0.001 to 2 and n and m have a value from zero to 100, with gaseous carbon dioxide.

**31.** The process of claim 30 wherein the alkoxide is selected from magnesium bis-butoxytriglycolate, magnesium bis-$\omega$-methoxypoly-ethoxide, zinc bis-butoxytriglycolate, zinc bis-$\omega$-methoxypoly-ethoxide, magnesium bis-hexyl-carbitolate, zinc bis-hexyl-carbitolate, ethoxymagnesium $\omega$-methoxypolyethoxide, methoxy-magnesiumethoxytriglycolate, ethoxymagnesium-ethoxytriglycolate, methoxymagnesiumbrn-toxytriglycolate and ethoxyzinc $\omega$-methoxypoly-ethoxide.

**Patentansprüche**

**1.** Verfahren zum Entsäuern von Cellulosematerial, das das Inkontaktbringen des Cellulosematerials mit einer Lösung in einem Kohlenwasserstoff oder Halogenkohlenwasserstoff oder Gemischen daraus umfaßt, die 0.01-1 mol/l eines oder mehrerer substituierter Metallalkoxide der Formel

$$X_yM^a(OR)_{a-y} \bullet (R^1OH)_x$$

enthält, in der:

(I) OR ein Rest ausgewählt aus 2-Alkoxyalkoxy- und $\omega$-Alkoxypolyalkoxyresten der Formel

$$[-OCH(R^2)CH_2(OCH(R^2)CH_2)_n)OR^3],$$

in der $R^2$ aus H und -$CH_3$ und $R^3$ aus Alkylresten mit 1 bis 18 Kohlenstoffatomen, Cycloalkylresten mit 3 bis 18 Kohlerstoffatomen und Aryl-, Arylalkyl- und Alkylarylresten mit 6 bis 18 Kohlenstoffatomen ausgewählt ist und n ein Wert von 1 bis 20 ist und in der -OR gegebenenfalls carboxyliert ist;
(II) X ein Rest ist, ausgewählt aus
(a) 2-Dialkylaminoalkoxy- und $\omega$-Dialkylaminopolyalkoxyresten der Formel

$$[-OCH(R^{2'})CH_2(OCH(R^{2'})CH_2)_mNR^{3'}_2],$$

in der $R^{2'}$ aus einem Wasserstoffatom und einer Methylgruppe ausgewählt, $R^{3'}$ aus Alkylresten mit 1 bis 18 Kohlenstoffatomen, Cycloalkylresten mit 3 bis 18 Kohlenstoffatomen und Aryl-, Arylalkyl- und Alkylarylresten mit 6 bis 18 Kohlenstoffatomen ausgewählt ist und m ein Wert von 1 bis 20 ist;
(b) einem Halogenatom, ausgewählt aus Chlor und Brom;
(c) einem organischen Rest -$R^4$, wobei $R^4$ ausgewählt ist aus der Gruppe, die aus Alkylresten mit 1 bis 18 Kohlenstoffatomen, Cycloakylresten mit 3 bis 18 Kohlenstoffatomen und Aryl-, Arylalkyl- und Alkylarylresten mit 6 bis 18 Kohlenstoffatomen besteht;
(d) einem Acyloxyrest der Formel [-O(O)CR⁴], in der $R^4$ die vorstehend angegebene Bedeutung hat;
(e) Alkoxypolyalkoxyresten der Formel

$[-OCH(R^{2'})CH_2(OCH(R^{2'})CH_2)_mOR^{3'}]$,

in der $R^{2'}$, $R^{3'}$ und m die vorstehend angegebene Bedeutung haben;

(f) einem Alkoxyrest $-OR^4$, in dem $R^4$ die vorstehend angegebene Bedeutung hat;

(III) M ein Metall ist, ausgewählt aus den Gruppen IIa und IIb des Periodensystems und Aluminium sowie Gemischen daraus;

(IV) $R^1OH$ eine Verbindung ist, in der $R^1O$ ein Rest ist, ausgewählt aus

(a) Alkoxyresten der Formel $R^{4'}O$, in der $R^{4'}$ die vorstehend für $R^4$ angegebene Bedeutung hat;

(b) 2-Alkoxyalkoxy- und $\omega$-Alkoxypolyalkoxyresten der Formel

$[-OCH(R^{2''})CH_2(OCH(R^{2''})CH_2)_oOR^{3''}]$,

in der $R^{2''}$, $R^{3''}$ und o die vorstehend für $R^2$, $R^3$ und n angegebene Bedeutung haben;

(c) 2-Dialkylaminoalkoxy- und $\omega$-Dialkylaminopolyalkoxyresten der Formel

$[-OCH(R^{2''})CH_2(OCH(R^{2''})CH_2)_oNR^{3''}_2]$

(V) a die Valenz des Metalls M ist;

(VI) y einen Wert zwischen null und eins hat und

(VII) x einen Wert von null bis zwei hat;

2. Verfahren gemäß Anspruch 1, das dadurch gekennzeichnet ist, daß mindestens eine der verwendeten Verbindungen eine Verbindung der Formel

$X_y M^a (OR)_{a-y} \bullet (R^1OH)_x$

ist, in der entweder $X_y$ oder $-OR$ carboxyliert ist und in der $R^1OH$, M, a, y und x die vorstehend angegebene Bedeutung haben
und

(I) OR ein Rest ist, ausgewählt aus 2-alkoxyalkoxy- und $\omega$-Alkoxypolyalkoxyresten der Formel

$[-OCH(R^2)CH_2(OCH(R^2)CH_2)_nOR^3]$,

in der $R^2$ aus H und $-CH_3$ und $R^3$ aus Alkylresten mit 1 bis 18 Kohlenstoffatomen, Cycloalkylresten mit 3 bis 18 Kohlenstoffatomen und Aryl-, Arylalkyl- und Alkylarylresten mit 6 bis 18 Kohlenstoffatomen ausgewählt ist und nein Wert von 1 bis 20 ist und

(II) X ein Rest ist, ausgewählt aus

(a) 2-Dialkylaminoalkoxy- und $\omega$-Dialkylaminopolyalkoxyresten der Formel

$[-OCH(R^{2'})CH_2(OCH(R^{2'})CH_2)_mNR^{3'}_2]$,

in der $R^{2'}$ aus einem Wasserstoffatom und einer Methylgruppe ausgewählt ist, $R^{3'}$ aus Alkylresten mit 1 bis 18 Kohlenstoffatomen, Cycloalkylresten mit 3 bis 18 Kohlenstoffatomen und Aryl-, Arylalkyl- und Alklarylresten mit 6 bis 18 Kohlenstoffatomen ausgewählt ist und m ein Wert von 1 bis 20 ist;

(b) einem Alkoxypolyalkoxyrest der Formel

$[-OCH(R^{2'})CH_2(OCH(R^{2'})CH_2)_mOR^{3'}]$,

in der $R^{2'}$ $R^{3'}$ und m die vorstehend angegebene Bedeutung haben und

(c) einem Alkoxyrest $-OR^4$, in dem $R^4$ die vorstehend angegebene Bedeutung hat.

3. Verfahren nach Anspruch 1 oder 2, bei dem die Lösung eine 0.02- bis 0.5-molare Lösung des substituierten Metallalkoxids ist.

4. Verfahren nach Anspruch 3, bei dem die Lösung eine 0.05-bis 0.25-molare Lösung des substituierten Metallalkoxids ist.

**5.** Verfahren gemäß einem der vorstehenden Ansprüche, bei dem das Cellulosematerial bei einem Druck zwischen 689.5 kPa und 6.895 MPa (100 und 1000 psi) mit einer Lösung substituierter Metallalkoxide und ihrer Carboxylderivate in Halogenkohlenwasserstoffen oder Kohlenwasserstoffen oder Gemischen daraus in Kontakt gebracht wird.

**6.** Verfahren nach einem der vorstehenden Ansprüche, bei dem die substituierten Metallalkoxide Metall-$\omega$-alkoxypolyalkoxide des Typs $(R^{3'}O(CH_2CH(R^{2'})O)_mCH_2CH(R^{2'})O)_yM^a(OCH(R^2)CH_2(OCH(R^2)CH_2)_nOR^3)_{a-y} \cdot (R^1OH)_x$ sind, wobei $R^2$ ein Wasserstoffatom oder eine Methylgruppe ist und $R^3$ und $R^{3'}$ gleiche oder verschiedene $C_1$-bis $C_{18}$-Alkylreste sind, $R^1$ ein $C_1$- bis $C_{18}$-Alkylrest oder ein $\omega$-Alkoxypolyalkoxy-alkylrest ist, a die Valenz des Metalls ist, n und m Werte von 1 bis 20 sind, x ein Wert von null bis 2 ist und y ein Wert zwischen 0.01 und 1 ist.

**7.** Verfahren nach einem der vorstehenden Ansprüche, bei dem die substituierten Metallalkoxide Alkylmetall-2-alkoxyalkoxy-und $\omega$-Alkoxypolyalkoxide $R^4yM(OCH(R^2)CH_2(OCH(R^2)CH_2)_nOR^3)_{a-y}$ sind, wobei $R^2$ aus einem Wasserstoffatom und einer Methylgruppe ausgewählt ist und $R^4$ und $R^3$ gleiche oder verschiedene $C_1$- bis $C_8$-Alkylreste sind, a die Valenz des Metalls ist, y ein Wert von 0.1 bis 1.0 ist und n ein Wert von 1 bis 20 ist.

**8.** Verfahren nach einem der vorstehenden Ansprüche, bei dem die substituierten Metallalkoxide aus der Gruppe der Magnesium-, Zink- und Aluminiumalkoxide und Gemischen daraus ausgewählt sind.

**9.** Verfahren nach einem der vorstehenden Ansprüche, bei dem die Carboxylderivate der substituierten Metallalkoxide carboxylierte Magnesium- und Zinkalkoxide sind und maximal eine Carbonatgruppe pro Metallatom besitzen.

**10.** Verfahren nach einem der vorstehenden Ansprüche, bei dem die carboxylierten, substituierten Metallalkoxide Alkoxymagnesium-, Alkoxyzink-2-alkoxyalkoxide und Magnesiurn-oder Zink-$\omega$-alkoxypolyalkoxide der Formel $(R^4O_yM(OC(O)OCH(R^2)CH_2(OCH(R^2)CH_2)_nOR^3)_{2-y} \cdot (R^1OH)_x$ sind, in der M aus Magnesium und Zink ausgewählt ist, $R^2$ ein Wasserstoffatom oder eine Methylgruppe ist, $R^3$ und $R^4$ gleiche oder verschiedene $C_1$- bis $C_{18}$-Alkylreste sind, $R^1$ ein $C_1$- bis $C_{18}$-Alkyl, 2-Alkoxyalkyl- oder $\omega$-Alkoxypolyalkoxyalkylrest ist, y ein Wert von 0.01 bis 1 ist, x ein Wert von null bis 2 ist und n ein Wert von 1 bis 20 ist.

**11.** Verfahren gemäß einem der vorstehenden Ansprüche, bei dem die Carboxylderivate der Metallalkoxide aus Magnesium-bis-butoxytriglycolat, Magnesium-bis-$\omega$-methoxypolyethoxid, Zink-bis-butoxytriglycolat, Zink-bis-$\omega$-methoxypolyethoxid, Magnesium-bis-hexylcarbitolat, Zink-bis-hexylcarbitolat, Ethoxymagnesium-$\omega$-methoxypolyethoxid, Methoxymagnesiumethoxytriglycolat, Ethoxymagnesiumethoxytriglycolat, Methoxymagnesiumbutoxytriglycolat und Ethoxyzink-$\omega$-methoxypolyethoxid ausgewählt sind, wobei jede Verbindung der Gruppe mit 0.2 bis 0.5 Moläquivalenten Butoxytriglycol komplexiert ist.

**12.** Verfahren gemäß einem der vorstehenden Ansprüche. bei dem das Cellulosematerial bei Atmosphärendruck mit normalenweise flüssigen Halogenkohlenwasserstoff- und Kohlenwasserstofflösungen von Metallalkoxiden und ihren Carboxylderivaten in Kontakt gebracht wird, wonach die Lösungen von dem Cellulosematerial entfernt werden.

**13.** Verfahren gemäß Anspruch 6, bei dem die Alkylmetällalkoxyalkoxide und Alkylmetall-$\omega$-alkoxypolyalkoxide aus Butylmagnesium-$\omega$-methoxypolyethoxid, Ethylzink-$\omega$-methoxy-polyethoxid, Butylaluminum-bis-$\omega$-methoxypolyethoxid und Gemischen daraus ausgewählt sind.

**14.** Verfahren gemäß Anspruch 1 oder 2, bei dem die carboxylierten, substituierten Metallalkoxide aus Verbindungen der Formeln

$$(R^4O)_yM(OC(O)OCH(R^2)CH_2(OCH(R^2)CH_2)_nOR^3)_{2-y} \cdot (R^1OH)_x$$

und

$$(R^4O(O)CO)_yM(OCH(R^2)CH_2(OCH(R^2)CH_2)_nOR^3)_{2-y} \cdot (R^1OH)_x$$

EP 0 388 448 B1

ausgewählt sind, in denen M aus Mg, Zn und Gemischen aus Mg und Zn ausgewählt ist, $R^3$ und $R^4$ $C_1$-$C_{18}$-Alkylreste sind, $R^2$ ein Wasserstoffatom oder eine Methylgruppe ist, $R^1$ aus $C_1$-$C_{18}$-Alkylresten und einem $R^{3''}O(CH_2CH(R^{2''})O)_oCH_2CH(R^{2''})$-Rest ausgewählt ist, in dem $R^{2''}$ und $R^{3''}$ die vorstehend für $R^2$ und $R^3$ angegebene Bedeutung haben, y ein Wert von 0.01 bis eins ist, n und o Werte von 1 bis 20 sind und x ein Wert von null bis zwei ist.

15. Verfahren gemäß Anspruch 1 oder 2, bei dem die carboxylierten, substituierten Metallalkoxide aus Verbindungen der Formeln

$$(R^{3'}O(CH_2CH(R^{2'})O)_mCH_2CH(R^{2'})O)_yM(OC(O)OCH(R^2)CH_2(OCH(R^2)CH_2)_nOR^3)_{2-y} \bullet (R^1OH)_x$$

und

$$(R^{3'}O(CH_2CH(R^{2'})O)_mCH_2CH(R^{2'})OC(O)O)_yM(OCH(R^2)CH_2(OCH(R^2)CH_2)_nOR^3)_{2-y} \bullet (R^1OH)_x$$

ausgewählt sind, in denen M aus Mg, Zn und Gemischen daraus ausgewählt ist, $R^2$ und $R^{2'}$ Wasserstoffatome oder Methylgruppen sind, $R^3$ und $R^{3'}$ gleiche oder verschiedene $C_1$-$C_{18}$-Alkylreste sind, $R^1$ aus $C_1$-$C_{18}$-Alkylresten und einem $R^{3''}O(CH_2CH(R^{2''})O)_oCH_2CH(R^{2''})$-Rest ausgewählt ist, in dem $R^{2''}$ und $R^{3''}$ die vorstehend für $R^2$ und $R^3$ angegebene Bedeutung haben, y ein Wert von 0.01 bis 1.0 ist, n, m und o Werte von 1 bis 18 sind und x ein Wert zwischen null und zwei ist.

16. Verfahren gemäß Anspruch 15, bei dem M Magnesium ist, n 2 ist, m 2 ist, $R^{3'}$ eine Butylgruppe ist, $R^3$ eine Butylgruppe ist, $R^2$, $R^{2'}$ und $R^{2''}$ Wasserstoffatome sind und x null ist.

17. Verfahren gemäß Anspruch 15, bei dem M Magnesium ist, m 6.4 ist, n 2 ist, $R^{3'}$ eine Methylgruppe ist, $R^3$ eine Butylgruppe ist, $R^2$, $R^{2'}$ und $R^{2''}$ Wasserstoffatome sind und x null ist.

18. Verfahren gemäß Anspruch 12, bei dem der Kohlenwasserstoff als Lösungsmittel aus der Gruppe ausgewählt ist, die aus Pentan, Hexan, Heptan, Benzol, Toluol, Cyclohexan, Ethan, Propan, Butan, Propylen und Gemischen daraus besteht.

19. Verfahren gemäß Anspruch 12, bei dem der Halogenkohlenwasserstoff aus der Gruppe ausgewählt ist, die aus 1,1,1-Trichlortrifluorethan, symmetrischem Tetrachlordifluorethan, Perchlorethylen, Chloroform, Methylchloroform, Chlortrifluormethan, Chlordifluorethan, 1,2-Dictuortetrafluorethan und Gemischen daraus besteht.

20. Zusammensetzung, die zur Verwendung in Verfahren nach den Ansprüchen 1 bis 19 geeignet ist und die ein Gemisch einer als (A) bezichneten Verbindung mit einer anderen, als (B) bezichneten Verbindung umfaßt, wobei (A) ein substituiertes Metallalkoxid der Formel

$$X_yM^a(OR)_{a-y} \bullet (R^1OH)_x$$

ist, in der
(I) OR ein Rest ist, ausgewählt aus 2-Alkoxyalkoxy- und ω- Alkoxypolyalkoxyresten der Formel

$$[-OCH(R^2)CH_2(OCH(R^2)CH_2)_nOR^3],$$

in der $R^2$ aus H und -$CH_3$ ausgewählt ist, $R^3$ aus Alkylresten mit 1 bis 18 Kohlenstoffatomen, Cycloalkylresten mit 3 bis 18 Kohlenstoffatomen und Aryl-, Arylalkyl- und Alkylarylresten mit 6 bis 18 Kohlenstoffatomen ausgewählt ist, n ein Wert von 1 bis 20 ist und in der -OR gegebenenfalls carboxyliert ist;
(II) X ein Rest ist, ausgewählt aus
(a) 2- Dialkylaminoalkoxy- und ω-Dialkylaminopolyalkoxyresten der Formel

$$[-OCH(R^{2'})CH_2(OCH(R^{2'})CH_2)_mNR^{3'}_2],$$

in der $R^{2'}$ aus einem Wasserstoffatom und einer Methylgruppe ausgewählt ist, $R^{3'}$ aus Alkylresten mit 1 bis 18 Kohlenstoffatomen, Cycloalkylresten mit 3 bis 18 Kohlenstoffatomen und Aryl-,

29

Arylalkyl- und Alkylarylresten mit 6 bis 18 Kohlenstoffatomen ausgewählt ist und m ein Wert von 1 bis 20 ist;

(b) einem Halogenatom, ausgewählt aus Chlor und Brom;

(c) einem organischen Rest $-R^4$, wobei $R^4$ ausgewählt ist aus der Gruppe, die aus Alkylresten mit 1 bis 18 Kohlenstoffatomen, Cycloalkylresten mit 3 bis 18 Kohlenstoffatomen und Aryl-, Arylalkyl- und Alkylarylresten mit 6 bis 18 Kohlenstoffatomen besteht;

(d) einem Acyloxyrest der Formel $[-O(O)CR^4]$, in der $R^4$ die vorstehend angegebene Bedeutung hat;

(e) Alkoxypolyalkoxyresten der Formel

$$[-OCH(R^{2'})CH_2(OCH(R^{2'})CH_2)_mOR^{3'}),$$

in der $R^{2'}$, $R^{3'}$ und m die vorstehend angegebene Bedeutung haben;

(f) einem Alkoxyrest $-OR^4$, in dem $R^4$ die vorstehend angegebene Bedeutung hat;

(III) M ein Metall ist, ausgewählt aus den Gruppen IIa und IIb des Periodensystems und Aluminium sowie Gemischen daraus;

(IV) $R^1OH$ eine Verbindung ist, in der $R^1O$ ein Rest ist, ausgewählt aus

(a) Alkoxyresten der Formel $R^{4'}O$, in der $R^{4'}$ die vorstehend für $R^4$ angegebeneBedeutung hat;

(b) 2-Alkoxyalkoxy- und $\omega$-Alkoxypolyalkoxyresten der Formel

$$[-OCH(R^{2''})CH_2(OCH(R^{2''})CH_2)_oOR^{3''}],$$

in der $R^{2''}$, $R^{3''}$ und o die vorstehend für $R^2$, $R^3$ und n angegebene Bedeutung haben;

(c) 2-Dialkylaminoalkoxy- und $\omega$-Dialkylaminopolyalkoxy-resten der Formel

$$[-OCH(R^{2''})CH_2(OCH(R^{2''})CH_2)_oNR^{3''}_2]$$

(V) a die Valenz des Metalls M ist;

(VI) y einen Wert zwischen null und eins hat;

(VII) x einen Wert von null bis zwei hat und

(B) ein carboxyliertes, substituiertes Metallalkoxid der Formel

$$X_yM^a(OR)_{a-y}\bullet(R^1OH)_x$$

ist, in der entweder $X_y$ oder $-OR$ carboxyliert ist und in der $R^1OH$ M, a, y und x die vorstehend angegebene Bedeutung haben

und

(I) OR ein Rest ist, ausgewählt aus 2-Alkoxyalkoxy- und $\omega$-Alkoxypolyalkoxyresten der Formel

$$[-OCH(R^2)CH_2(OCH(R^2)CH_2)_nOR^3],$$

in der $R^2$ aus H und $-CH_3$ und $R^3$ aus Alkylresten mit 1 bis 18 Kohlenstoffatomen, Cycloalkylresten mit 3 bis 18 Kohlenstoffatomen und Aryl-, Arylalky- und Alkylarylresten mit 6 bis 18 Kohlensteffatomen ausgewählt ist und n ein Wert von 1 bis 20 ist und

(II) X ein Rest ist, ausgewählt aus

(a) 2-Dialkylaminoalkoxy- und $\omega$-Dialkylaminopolyalkoxy-resten der Formel

$$[-OCH(R^{2'})CH_2(OCH(R^{2'})CH_2)_mNR^{3'}_2],$$

in der $R^{2'}$ aus einem Wasserstoffatom und einer Methylgruppe ausgewählt ist, $R^{3'}$ aus Alkylresten mit 1 bis 18 Kohlenstoffatomen, Cycloalkylresten mit 3 bis 18 Kohlenstoffatomen und Aryl-, Arylalkyl- und Alkylarylresten mit 6 bis 18 Kohlenstoffatomen ausgewählt ist und m ein Wert von 1 bis 20 ist;

(b) einem Alkoxypolyalkoxyrest der Formel

$$[-OCH(R^{2'})CH_2(OCH(R^{2'})CH_2)_mOR^{3'}],$$

in der $R^{2'}$, $R^{3'}$ und m die vorstehend angegebene Bedeutung haben und

(c) einem Alkoxyrest $-OR^4$, in dem $R^4$ die vorstehend angegebene Bedeutung hat.

21. Zusammensetzung gemäß Anspruch 20, die ein Gemisch von Verbindungen umfaßt, die aus Verbindungen der Formeln

$$(R^4O)_yM(OC(O)OCH(R^2)CH_2(OCH(R^2)CH_2)_nOR^3)_{2-y} \bullet (R^1OH)_x$$

und

$$(R^4O(O)CO)_yM(OCH(R^2)CH_2(OCH(R^2)CH_2)_nOR^3)_{2-y} \bullet (R^1OH)_x$$

ausgewählt sind, in denen M aus Mg, Zn und Gemischen aus Mg und Zn ausgewählt ist, n ein Wert von 1 bis 20 ist, $R^3$ und $R^4$ $C_1$-$C_{18}$-Kohlenwasserstoflreste sind, $R^2$ ein Wasserstoffatom oder eine Methylgruppe ist und $R^1$ gleich oder verschieden von $R^4$ und/oder $R^{3''}O(CH_2CH(R^{2''})O)_nCH_2CH(R^{2''})$ ist, y ein Wert von 0.01 bis eins ist und x ein Wert von null bis zwei ist.

22. Zusammensetzung nach Anspruch 21, in der M Magnesium ist, $R^3$ und $R^4$ aus Methyl-, Ethyl- und Butylgruppen ausgewählt sind, $R^2$ ein Wasserstoffatom ist, $R^1$ aus Methyl-, Ethyl- und Butylgruppen und einem $R^{3''}O(CH_2CHO)_2CH_2CH_2$-Rest ausgewählt ist, in dem $R^{3''}$ aus Methyl-, Ethyl- und Butylgruppen ausgewählt ist, n zwei ist, y eins ist und x null bis zwei ist.

23. Zusammensetzung nach Anspruch 22, in der n 6.4 ist, $R^3$ eine Methylgruppe ist, $R^4$ eine Ethylgruppe ist, $R^1$ $C_4H_9O(CH_2CH_2O)_2CH_2CH_2$- ist, y eins ist und x 0.2 bis 0.5 ist.

24. Zusammensetzung nach Anspruch 20, in der $R^2$ eine Ethylgruppe ist, $R^4$ aus einer Methylgruppe und $C_4H_9O(CH_2CH_2O)CH_2CH_2$- ausgewählt ist und x 1.5 ist.

25. Zusammensetzung nach Anspruch 21, in der M Magnesium ist, n 2 ist, $R^4$ eine Ethylgruppe ist, $R^3$ eine Butylgruppe ist, $R^2$ ein Wasserstoffatom ist, $R^1$ $C_4H_9O(CH_2CH_2O)_2CH_2CH_2$- ist und x 0.5 ist.

26. Zusammensetzung nach Anspruch 21, in der M Magnesium ist, n 6.4 ist, $R^3$ eine Methylgruppe ist, $R^4$ eine Ethylgruppe ist, $R^2$ ein Wasserstoffatom ist, $R^1$ $C_4H_9O(CH_2CH_2O)_2CH_2CH_2$- ist und x 0.25 ist.

27. Zusammensetzung gemäß Anspruch 20, die ein Gemisch von Verbindungen umfaßt, die aus Verbindungen der Formeln

$$(R^{3'}O(CH_2CH(R^{2'})O)_mCH_2CH(R^{2'}O)_yM(OC(O)OCH(R^2)CH_2(OCH(R^2)CH_2)_nOR^3)_{2-y} \bullet (R^1OH)_x$$

und

$$(R^{3'}O(CH_2CH(R^{2'})O)_mCH_2CH(R^{2'}OC(O)O)_yM(OCH(R^2)CH_2(OCH(R^2)CH_2)_nOR^3)_{2-y} \bullet (R^1OH)_x$$

ausgewählt sind, in denen M aus Mg, Zn und Gemischen daraus ausgewählt ist, n und m Werte von 1 bis 20 sind, $R^2$ und $R^{2'}$ Wasserstoffatome oder Methylgruppen sind, $R^3$ und $R^{3'}$ $C_1$-$C_{18}$-Alkylreste sind, $R^1$ $R^{3''}O(CH_2CH_2O)_oCH_2CH_2$- ist, wobei $R^{3''}$ die vorstehend für $R^3$ angegebene und o die vorstehend für n angegebene Bedeutung hat, y ein Wert von 0.01 bis eins ist und x ein Wert zwischen null und zwei ist.

28. Zusammensetzung nach Anspruch 27, in der M Magnesium ist, m 6.4 ist, n 2 ist, $R^{3'}$ eine Methylgruppe ist, $R^3$ eine Butylgruppe ist, $R^2$ und $R^{2'}$ Wasserstoffatome sind und x null ist.

29. Zusammensetzung nach Anspruch 27, in der M Magnesium ist, n 2 ist, m 2 ist, $R^{3'}$ eine Butylgruppe ist, $R^3$ eine Butylgruppe ist, $R^2$ und $R^{2'}$ Wasserstoffatome sind und x null ist.

30. Verfahren zur Herstellung flüssiger, carboxylierter Metall-2-alkoxy- und $\omega$-alkoxypolyalkoxide mit niedriger Viskosität, das die Umsetzung eines Reaktanten, der aus substituierten Metall-2-alkoxyalkoxy- und Metall-$\omega$-alkoxypolyalkoxiden der Formeln

$$(R^4 O)_y M^a (OCH(R^2)CH_2 (OCH(R^2)CH_2)_n OR^3)_{a-y} \bullet (R^1 OH)_x$$

und

$$(R^{3'} O(CH_2 CH(R^{2'})O)_m CH_2 CH(R^{2'})O)_y M^a (OCH(R^2)CH_2 (OCH(R^2)CH_2)_m OR^3)_{a-y} \bullet (R^1 OH)_x$$

ausgewählt ist, in denen M ein aus Magnesium, Zink und Gemischen aus Magnesium und Zink ausgewähltes Metall ist, $R^2$ und $R^{2'}$ aus einem Wasserstoffatom und einer Methylgruppe ausgewählt sind, $R^1$, $R^3$ und $R^{3'}$ voneinander unabhängig aus Alkylresten mit 1 bis 12 Kohlenstoffatomen ausgewählt sind, y einen Wert von 0.01 bis 1 hat, x einen Wert von 0.001 bis 2 hat und n und m einen Wert von null bis 100 haben, mit gasförmigem Kohlendioxid in einem aus flüssigen Kohlenwasserstoffen und flüssigen Halogenkohlenwasserstoffen ausgewählten Lösungsmittel umfaßt.

31. Verfahren nach Anspruch 30, bei dem das Alkoxid aus Magnesium-bis-butoxytriglycolat, Magnesium-bis-ω-methoxypolyethoxid, Zink-bis-butoxytriglycolat, Zink-bis-ω-methoxypolyethoxid, Magnesium-bis-hexylcarbitolat, Zink-bis-hexylcarbitolat, Ethoxymagnesium-ω-methoxypolyethoxid, Methoxymagnesiumethoxytriglycolat, Ethoxymagnesiumethoxytriglycolat, Methoxymagnesiumbutoxytriglycolat und Ethoxyzink-ω-methoxypolyethoxid ausgewählt ist.

**Revendications**

1. Procédé pour désacidifier des matières cellulosiques, comprenant la mise en contact de la matière cellulosique avec une solution, dans un hydrocarbure ou un halogénocarbure ou des mélanges de ceux-ci, contenant 0,01 à 1 mole/ℓ d'un ou plusieurs alcoxydes métalliques substitués ayant la formule

$$X_y M^a (OR)_{a-y} . (R^1 OH)_x$$

où :
(I) OR est un groupe choisi parmi les groupes 2-alcoxyalcoxy et ω-alcoxypolyalcoxy de formule

$$[-OCH(R^2)CH_2 (OCH(R^2)CH_2)_n OR^3]$$

où $R^2$ est choisi parmi H et -$CH_3$ et $R^3$ est choisi parmi les groupes alkyle de 1 à 18 atomes de carbone, les groupes cycloalkyle de 3 à 18 atomes de carbone, et les groupes aryle, arylalkyle et alkylaryle de 6 à 18 atomes de carbone, et n est une valeur de 1 à 20, et dans laquelle -OR est éventuellement carbonaté ;
(II) X est un groupe choisi parmi
(a) les groupes 2-dialkylaminoalcoxy et ω-dialkylaminopolyalcoxy de formule

$$[-OCH(R^{2'} CH_2 (OCH(R^{2'})CH_2)_m NR^{3'}_2]$$

où $R^{2'}$ est choisi parmi l'hydrogène et le radical méthyle, $R^{3'}$ est choisi parmi les groupes alkyle de 1 à 18 atomes de carbone, les groupes cycloalkyle de 3 à 18 atomes de carbone et les groupes aryle, arylalkyle et alkylaryle de 6 à 18 atomes de carbone, et m est une valeur de 1 à 20 ;
(b) un halogène choisi parmi le chlore et le brome ;
(c) un groupe organique -$R^4$ où $R^4$ est choisi dans le groupe constitué des groupes alkyle contenant 1 à 18 atomes de carbone, des groupes cycloalkyle contenant 3 à 18 atomes de carbone, et les groupes aryle, arylalkyle et alkylaryle contenant 6 à 18 atomes de carbone ;
(d) un groupe acyloxy de formule [-O(O)C$R^4$] où $R^4$ a la signification donnée ci-dessus ;
(e) les groupes alcoxypolyalcoxy de formule

$$[-OCH(R^{2'})CH_2 (OCH(R^{2'})CH_2)_m OR^{3'}]$$

où $R^{2'}$, $R^{3'}$ et m ont les significations données ci-dessus ;
(f) un groupe alcoxy -$OR^4$ où $R^4$ a les significations données ci-dessus ;
(III) M est un métal choisi parmi les groupes IIa et IIb du tableau périodique et l'aluminium et les mélanges de ceux-ci ;

(IV) $R^1OH$ est un composé dans lequel $R^1O$ est un groupe choisi parmi
(a) les groupes alcoxy de formule $R^{4'}O$ où $R^{4'}$ a les significations décrites ci-dessus pour $R^4$ ;
(b) les groupes 2-alcoxyalcoxy et $\omega$-alcoxypolyalcoxy de formule

$$[-OCH(R^{2''})CH_2(OCH(R^{2''})CH_2)_oOR^{3''}]$$

où $R^{2''}$, $R^{3''}$ et o ont les significations données ci-dessus pour $R^2$, $R^3$ et n ;
(c) les groupes 2-dialkylaminoalcoxy et $\omega$-dialkylaminopolyalcoxy de formule

$$[-OCH(R^{2''})CH_2(OCH(R^{2''})CH_2)_oNR^{3''}{}_2]$$

(V) a est la valence du métal M ;
(VI) y a une valeur entre zéro et un ; et
(VII) x a une valeur de zéro à deux.

2. Procédé selon la revendication 1, **caractérisé en ce que** au moins l'un des composés utilisés est un composé de formule $X_yM^a(OR)_{a-y}.(R^1OH)_x$ dans laquelle $X_y$ ou $-OR$ est carbonaté, où $R^1OH$, M, a, y et x ont les significations données ci-dessus ; et
(I) OR est un groupe choisi parmi les groupes 2-alcoxyalcoxy et $\omega$-alcoxypolyalcoxy de formule

$$[-OCH(R^2)CH_2(OCH(R^2)CH_2)_nOR^3]$$

où $R^2$ est choisi parmi H et $-CH_3$ et $R^3$ est choisi parmi les groupes alkyle de 1 à 18 atomes de carbone, les groupes cycloalkyle de 3 à 18 atomes de carbone, et les groupes aryle, arylalkyle et alkylaryle de 6 à 18 atomes de carbone, et n est une valeur de 1 à 20 ; et
(II) X est un groupe choisi parmi
(a) les groupes 2-dialkylaminoalcoxy et $\omega$-dialkylaminopolyalcoxy de formule

$$[OCH(R^{2'})CH_2(OCH(R^{2'})CH_2)_mNR^{3'}{}_2]$$

où $R^{2'}$ est choisi parmi l'hydrogène et le radical méthyle, $R^{3'}$ est choisi parmi les groupes alkyle de 1 à 18 atomes de carbone, les groupes cycloalkyle de 3 à 18 atomes de carbone et les groupes aryle, arylalkyle et alkylaryle de 6 à 18 atomes de carbone, et m est une valeur de 1 à 20 ;
(b) un groupe alcoxypolyalcoxy de formule

$$[-OCH(R^{2'})CH_2(OCH(R^{2'})CH_2)_mOR^{3'}]$$

où $R^{2'}$, $R^{3'}$ et m ont les significations données ci-dessus ; et
(c) un groupe alcoxy $-OR^4$ où $R^4$ a la signification donnée ci-dessus.

3. Procédé selon la revendication 1 ou 2, dans lequel la solution est une solution 0,02 à 0,5 molaire de l'alcoxyde métallique substitué.

4. Procédé selon la revendication 3, dans lequel la solution est une solution 0,05 à 0,25 molaire de l'alcoxyde métallique substitué.

5. Procédé selon l'une quelconque des revendications précédentes, dans lequel lesdites matières cellulosiques sont mises en contact avec une solution, dans un halogénocarbure ou un hydrocarbure ou des mélanges de ceux-ci, d'alcoxydes métalliques substitués et de leurs dérivés carbonatés sous une pression entre 689,5 kPa et 6,895 MPa (100 et 1000 psi).

6. Procédé selon l'une quelconque des revendications précédentes, dans lequel lesdits alcoxydes métalliques substitués sont des $\omega$-alcoxypolyalcoxydes métalliques du type

$$(R^{3'}O(CH_2CH(R^{2'})O)_mCH_2CH(R^{2'})O)_yM^a(OCH(R^2)CH_2(OCH(R^2)CH_2)_n-OR^3)_{a-y}.(R^1OH)_x$$

où $R^2$ est un hydrogène ou un groupe méthyle, et $R^3$ et $R^{3'}$ sont des groupes alkyle en $C_1$ à $c_{18}$

identiques ou différents, $R^1$ est un groupe alkyle en $C_1$ à $C_{18}$ ou un groupe $\omega$-alcoxypolyalcoxyalkyle, a est la valence du métal, n et m sont des valeurs de 1 à 20, x est une valeur de zéro à 2 et y est une valeur entre 0,01 et 1.

7. Procédé selon l'une quelconque des revendications précédentes, dans lequel lesdits alcoxydes métalliques substitués sont des 2-alcoxyalcoxy- et $\omega$-alcoxy-polyalcoxydes d'alkylmétal $R^4{}_yM(OCH(R^2)$-$CH_2(OCH(R^2)CH)_n$-$OR^3)_{a-y}$, où $R^2$ est choisi parmi l'hydrogène et un groupe méthyle, et $R^4$ et $R^3$ sont des groupes alkyle en $C_1$ à $C_{18}$ identiques ou différents, a est la valence du métal, y est une valeur de 0,1 à 1,0, et n est une valeur de 1 à 20.

8. Procédé selon l'une quelconque des revendications précédentes, dans lequel les alcoxydes métalliques substitués sont choisis dans le groupe des alcoxydes de magnésium, de zinc, d'aluminium et des mélanges de ceux-ci.

9. Procédé selon l'une quelconque des revendications précédentes, dans lequel lesdits dérivés carbonatés desdits alcoxydes métalliques substitués sont des alcoxydes de magnésium et de zinc carbonatés et possèdent un maximum d'un groupe carbonate par atome de métal.

10. Procédé selon l'une quelconque des revendications précédentes, dans lequel lesdits alcoxydes métalliques substitués carbonatés sont des 2-alcoxyalcoxydes d'alcoxymagnésium-, d'alcoxyzinc, et des $\omega$-alcoxypolyalcoxydes de magnésium ou de zinc, de formule $(R^4O)_yM(OC(O)OCH(R^2)CH_2(OCH$-$(R^2)CH_2)_nOR^3{}_{2-y}.(R^1OH)_x$, où M est choisi parmi le magnésium et le zinc, $R^2$ est de l'hydrogène ou un groupe méthyle, $R^3$ et $R^4$ sont des groupes alkyle en $C_1$ à $C_{18}$ identiques ou différents, $R^1$ est un groupe alkyle en $C_1$ à $C_{18}$, un groupe 2-alcoxyalkyle ou $\omega$-alcoxypolyalcoxyalkyle, y est une valeur de 0,01 à 1, x est une valeur de zéro à 2, et n est une valeur de 1 à 20.

11. Procédé selon l'une quelconque des revendications précédentes, dans lequel lesdits dérivés carbonatés desdits alcoxydes métalliques sont choisis parmi le bis-butoxytriglycolate de magnésium, le bis-$\omega$-méthoxypolyéthoxyde de magnésium, le bis-butoxytriglycolate de zinc, le bis-$\omega$-méthoxypolyéthoxyde de zinc, le bis-hexylcarbitolate de magnésium, le bis-hexylcarbitolate de zinc, le $\omega$-méthoxypolyéthoxyde d'éthoxymagnésium, l'éthoxytriglycolate de méthoxymagnésium, l'éthoxytriglycolate d'éthoxymagnésium, le butoxytriglycolate de méthoxymagnésium et le $\omega$-méthoxypolyéthoxyde d'éthoxyzinc, chaque élément du groupe complexé avec 0,2 à 0,5 équivalent molaire de butoxytriglycol.

12. Procédé selon l'une quelconque des revendications précédentes, dans lequel lesdites matières cellulosiques sont mises en contact avec des solutions halogénocarbonées et hydrocarbonées normalement liquides d'alcoxydes métalliques et de leurs dérivés carbonatés, à pression atmosphérique, puis lesdites solutions sont éliminées desdites matières cellulosiques.

13. Procédé selon la revendication 6, dans lequel lesdits alcoxyalcoxydes d'alkylmétal et $\omega$-alcoxypolyalcoxydes d'alkylmétal sont choisis parmi le $\omega$-méthoxypolyéthoxyde de butylmagnésium, le $\omega$-méthoxypolyéthoxyde d'éthylzinc, le bis-$\omega$-méthoxypolyéthoxyde de butylaluminium et les mélanges de ceux-ci.

14. Procédé selon la revendication 1 ou 2, dans lequel les alcoxydes métalliques substitués carbonatés sont choisis parmi les composés ayant les formules

$$(R^4O)_yM(OC(O)OCH(R^2)CH_2(OCH(R^2)CH_2)_nOR^3)_{2-y}.(R^1OH)_x$$

et

$$(R^4O(O)CO)_yM(OCH(R^2)CH_2(OCH(R^2)CH_2)_nOR^3)_{2-y}.(R^1OH)_x,$$

où M est choisi parmi Mg, Zn et les mélanges de Mg et de Zn, $R^3$ et $R^4$ sont des groupes alkyle en $C_1$ à $C_{18}$, $R^2$ est de l'hydrogène ou un radical méthyle, $R^1$ est choisi parmi les groupes alkyle en $C_1$ à $C_{18}$ et un groupe $R^{3"}O(CH_2CH(^{2"})O)_oCH_2CH(R^{2"})$ où $R^{2"}$ et $R^{3"}$ ont les significations données ci-dessus pour $R^2$ et $R^3$, y est une valeur de 0,01 à un, n et o sont des valeurs de 1 à 20 et x est une valeur de zéro à deux.

**15.** Procédé selon la revendication 1 ou 2, dans lequel les alcoxydes métalliques substitués carbonatés sont choisis parmi les composés ayant les formules

$$(R^{3'}O(CH_2CH(R^{2'})O)_mCH_2CH(R^{2'})O)_yM(OC(O)OCH(R^2)CH_2(OCH\,(R^2)CH_2)_nOR^3)_{2-y}.(R^1OH)_x \text{ et}$$
$$(R^{3'}O(CH_2CH(R^{2'})O)_mCH_2CH(R^{2'})OC(O)O)_yM(OCH(R^2)CH_2(OCH\,(R^2)CH_2)_nOR^3)_{2-y}.(R^1OH)_x$$

où M est choisi parmi Mg, Zn et les mélanges de ceux-ci, $R^2$ et $R^{2'}$ sont un hydrogène ou un radical méthyle, $R^3$ et $R^{3'}$ sont des groupes alkyle en $C_1$ à $C_{18}$ identiques ou différents, $R^1$ est choisi parmi les groupes alkyle en $C_1$ à $C_{19}$ et un groupe $R^{3''}O(CH_2CH(R^{2''})O)_oCH_2CH(R^{2''})$ où $R^{3''}$ et $R^{2''}$ ont les significations données ci-dessus pour $R^2$ et $R^3$, y est une valeur de 0,01 à 1,0, n, m et o sont des valeurs de 1 à 18 et x est une valeur entre zéro et deux.

**16.** Procédé selon la revendication 15, dans lequel M est du magnésium, n est 2, m est 2, $R^{3'}$ est un radical butyle, $R^3$ est un radical butyle, $R^2$, $R^{2'}$ et $R^{2''}$ sont de l'hydrogène et x est zéro.

**17.** Procédé selon la revendication 15, dans lequel M est du magnésium, m est 6,4, n est 2, $R^{3'}$ est un radical méthyle, $R^3$ est un radical butyle, $R^2$, $R^{2'}$ et $R^{2''}$ sont un hydrogène et x est zéro.

**18.** Procédé selon la revendication 12, dans lequel le solvant hydrocarboné est choisi dans le groupe constitué du pentane, de l'hexane, de l'heptane, du benzène, du toluène, du cyclohexane, de l'éthane, du propane, du butane, du propylène et des mélanges de ceux-ci.

**19.** Procédé selon la revendication 12, dans lequel l'halogénocarbure est choisi dans le groupe constitué du 1,1,1-trichlorotrifluoroéthane, du tétrachlorodifluoroéthane symétrique, du perchloréthylène, du chloroforme, du méthylchloroforme, du chlorotrifluorométhane, du chlorodifluoroéthane, du 1,2-dichlorotétrafluoroéthane et des mélanges de ceux-ci.

**20.** Composition appropriée pour l'utilisation dans le procédé de l'une des revendications 1 à 19, comprenant un mélange d'un composé défini par (A) avec un autre composé défini par (B), où (A) est un alcoxyde métallique substitué ayant la formule

$$X_yM^a(OR)_{a-y}.(R^1OH)_x$$

(I) OR est un groupe choisi parmi les groupes 2-alcoxyalcoxy et $\omega$-alcoxypolyalcoxy de formule

$$[-OCH(R^2)CH_2(OCH(R^2)CH_2)_nOR^3]$$

où $R^2$ est choisi parmi H et $-CH_3$ et $R^3$ est choisi parmi les groupes alkyle de 1 à 18 atomes de carbone, les groupes cycloalkyle de 3 à 18 atomes de carbone, et les groupes aryle, arylalkyle et alkylaryle de 6 à 18 atomes de carbone, et n est une valeur de 1 à 20, et dans laquelle -OR est éventuellement carbonaté ;
(II) X est un groupe choisi parmi
  (a) les groupes 2-dialkylaminoalcoxy et $\omega$-dialkylaminopolyalcoxy de formule

$$[-OCH(R^{2'})CH_2(OCH(R^{2'})CH_2)_mNR^{3'}_2]$$

où $R^{2'}$ est choisi parmi l'hydrogène et le radical méthyle, $R^{3'}$ est choisi parmi les groupes alkyle de 1 à 18 atomes de carbone, les groupes cycloalkyle de 3 à 18 atomes de carbone et les groupes aryle, arylalkyle et alkylaryle de 6 à 18 atomes de carbone, et m est une valeur de 1 à 20 ;
  (b) un halogène choisi parmi le chlore et le brome ;
  (c) un groupe organique $-R^4$ où $R^4$ est choisi dans le groupe constitué des groupes alkyle contenant 1 à 18 atomes de carbone, des groupes cycloalkyle contenant 3 à 18 atomes de carbone, et des groupes aryle, arylalkyle; et alkylaryle contenant 6 à 18 atomes de carbone ;
  (d) un groupe acyloxy de formule $[-O(O)CR^4]$ où $R^4$ a la signification donnée ci-dessus ;
  (e) les groupes alcoxypolyalcoxy de formule

$$[-OCH(R^{2'})CH_2(OCH(R^{2'})CH_2)_mOR^{3'}]$$

où $R^{2'}$, $R^{3'}$ et m ont les significations données ci-dessus ;

(f) un groupe alcoxy $-OR^4$ où $R^4$ a les significations données ci-dessus ;

(III) M est un métal choisi parmi les groupes IIa et IIb du tableau périodique et l'aluminium et les mélanges de ceux-ci ;

(IV) $R^1OH$ est un composé dans lequel $R^1O$ est un groupe choisi parmi

(a) les groupes alcoxy de formule $R^{4'}O$ où $R^{4'}$ a les significations décrites ci-dessus pour $R^4$ ;

(b) les groupes 2-alcoxyalcoxy et $\omega$-alcoxypolyalcoxy de formule

$$[-OCH(R^{2''})CH_2(OCH(R^{2''})CH_2)_oOR^{3''}]$$

où $R^{2''}$, $R^{3''}$ et o ont les significations données ci-dessus pour $R^2$, $R^3$ et n ;

(c) les groupes 2-dialkylaminoalcoxy et $\omega$-dialkylaminopolyalcoxy de formule

$$[-OCH(R^{2''})CH_2(OCH(R^{2''})CH_2)_oNR^{3''}_2]$$

(V) a est la valence du métal M ;

(VI) y a une valeur entre zéro et un ; et

(VII) x a une valeur de zéro à deux ; et

(B) est un alcoxyde métallique substitué carbonaté ayant la formule

$$X_yM^a(OR)_{a-y}.(R^1OH)_x$$

dans laquelle $X_y$ ou $-OR$ est carbonaté, où $R^1OH$, M, a, y et x ont les significations données ci-dessus ; et

(I) OR est un groupe choisi parmi les groupes 2-alcoxyalcoxy et $\omega$-alcoxypolyalcoxy de formule

$$[-OCH(R^2)CH_2(OCH(R^2)CH_2)_nOR^3]$$

où $R^2$ est choisi parmi H et $-CH_3$ et $R^3$ est choisi parmi les groupes alkyle de 1 à 18 atomes de carbone, les groupes cycloalkyle de 3 à 18 atomes de carbone, et les groupes aryle, arylalkyle et alkylaryle de 6 à 18 atomes de carbone, et n est une valeur de 1 à 20 ; et

(II) X est un groupe choisi parmi

(a) les groupes 2-dialkylaminoalcoxy et $\omega$-dialkylaminopolyalcoxy de formule

$$[-OCH(R^{2'})CH_2(OCH(R^{2'})CH_2)_mNR^{3'}_2]$$

où $R^{2'}$ est choisi parmi l'hydrogène et le radical méthyle, $R^{3'}$ est choisi parmi les groupes alkyle de 1 à 18 atomes de carbone, les groupes cycloalkyle de 3 à 18 atomes de carbone et les groupes aryle, arylalkyle et alkylaryle de 6 à 18 atomes de carbone, et m est une valeur de 1 à 20 ;

(b) un groupe alcoxypolyalcoxy de formule

$$[-OCH(R^{2'})CH_2(OCH(R^{2'})CH_2)_mOR^{3'}]$$

où $R^{2'}$, $R^{3'}$ et m ont les significations données ci-dessus ; et

(c) un groupe alcoxy $-OR^4$ où $R^4$ a la signification donnée ci-dessus.

**21.** Composition selon la revendication 20, comprenant un mélange de composés choisis parmi les composés de formule

$$(R^4O)_yM(OC(O)OCH(R^2)CH_2(OCH(R^2)CH_2)_nOR^3)_{2-y}.(R^1OH)_x$$

et

$$(R^4O(O)CO)_yM(OCH(R^2)CH_2(OCH(R^2)CH_2)_nOR^3)_{2-y}.(R^1OH)_x,$$

où M est choisi parmi Mg, Zn et les mélanges de Mg et de Zn, n est une valeur de 1 à 20, $R^3$ et $R^4$ sont des groupes hydrocarbyle en $C_1$ à $C_{18}$, $R^2$ est de l'hydrogène ou un radical méthyle, et $R^1$ est le

même ou différent $R^4$ et/ou $R^{3''}O(CH_2CH(R^{2''})O)_nCH_2CH(R^{2''})$, y est une valeur de 0,01 à un et x est une valeur de zéro à deux.

22. Composition selon la revendication 21, dans laquelle M est le magnésium, $R^3$ et $R^4$ sont choisis parmi les radicaux méthyle, éthyle et butyle, $R^2$ est de l'hydrogène et $R^1$ est choisi parmi les radicaux méthyle, éthyle, butyle et $R^{3''}O(CH_2CHO)_2CH_2CH_2$- où $R^{3''}$ est choisi parmi les groupes méthyle, éthyle et butyle, n est deux, y est un et x est zéro à deux.

23. Composition selon la revendication 22, dans laquelle n est 6,4, $R^3$ est un radical méthyle, $R^4$ est un radical éthyle, $R^1$ est $C_4H_9O(CH_2CH_2O)_2CH_2CH_2$-et y est un et x est 0,2 à 0,5.

24. Composition selon la revendication 20, dans laquelle $R^2$ est un radical éthyle, $R^4$ est choisi parmi les radicaux méthyle et $C_4H_9O(CH_2CH_2O)CH_2CH_2$-et x est 1,5.

25. Composition selon la revendication 21, dans laquelle M est le magnésium, n est 2, $R^4$ est un radical éthyle, $R^3$ est un radical butyle, $R^2$ est de l'hydrogène, $R^1$ est $C_4H_9O(CH_2CH_2O)_2CH_2CH_2$- et x est 0,5.

26. Composition selon la revendication 21, dans laquelle M est le magnésium, n est 6,4, $R^3$ est un radical méthyle, $R^4$ est un radical éthyle, $R^2$ est de l'hydrogène, $R^1$ est $C_4H_9O(CH_2CH_2O)_2CH_2CH_2$- et x est 0,25.

27. Composition selon la revendication 20, comprenant un mélange de composés choisis parmi les composés de formule

$$(R^{3'}O(CH_2CH(R^{2'})O)_mCH_2CH(R^{2'})O)_yM(OC(O)OCH(R^2)CH_2(OCH(R^2)CH_2)_nOR^3)_{2-y}\cdot(R^1OH)_x$$

et

$$(R^{3'}O(CH_2CH(R^{2'})O)_mCH_2CH(R^{2'})OC(O)O)_yM(OCH(R^2)CH_2(OCH(R^2)CH_2)_nOR^3)_{2-y}\cdot(R^1OH)_x$$

où M est choisi parmi Mg, Zn et les mélanges de ceux-ci, n et m sont des valeurs de 1 à 20, $R^2$ et $R^{2'}$ sont un hydrogène ou un radical méthyle, $R^3$ et $R^{3'}$ sont des groupes alkyle en $C_1$ à $C_{18}$, $R^1$ est $R^{3''}O$-$(CH_2CH_2O)_oCH_2CH_2$-, où $R^{3''}$ a la signification auparavant attribuée à $R^3$, et o la signification auparavant attribuée à n, y est une valeur de 0,01 à un, et x est une valeur entre zéro et deux.

28. Composition selon la revendication 27, dans laquelle M est le magnésium, m est 6,4, n est 2, $R^{3'}$ est un radical méthyle, $R^3$ est un radical butyle, $R^2$ et $R^{2'}$ sont de l'hydrogène et x est zéro.

29. Composition selon la revendication 27, dans laquelle M est le magnésium, n est 2, m est 2, $R^{3'}$ est un radical butyle, $R^3$ est un radical butyle, $R^2$ et $R^{2'}$ sont de l'hydrogène et x est zéro.

30. Procédé pour préparer des 2-alcoxy- et $\omega$-alcoxypolyalcoxydes métalliques carbonatés fluides, de basse viscosité, comprenant la réaction, dans un solvant choisi parmi les solvants hydrocarbonés liquides et les solvants halogénocarbonés liquides, d'un réactif choisi parmi les 2-alcoxyalcoxy-métalliques et $\omega$-alcoxypolyalcoxydes métalliques substitués, de formule

$$(R^4O)_yM^a(OCH(R^2)CH_2(OCH(R^2)CH_2)_nOR^3)_{a-y}\cdot(R^1OH)_x$$

et

$$(R^{3'}O(CH_2CH(R^{2'})O)_mCH_2CH(R^{2'})O)_yM^a(OCH(R^2)CH(OCH(R^2)CH_2)_mOR^3)_{a-y}\cdot(R^1OH)_x$$

où M est un métal choisi parmi le magnésium, le zinc et les mélanges de Mg et Zn, $R^2$ et $R^{2'}$ sont choisis parmi l'hydrogène et un radical méthyle, $R^1$, $R^3$ et $R^{3'}$ sont indépendamment choisis parmi les groupes alkyle contenant 1 à 12 atomes de carbone, y a une valeur de 0,01 à 1, x est une valeur de 0,001 à 2, et n et m ont une valeur de zéro à 100, avec du dioxyde de carbone gazeux.

**31.** Procédé selon la revendication 30, dans lequel l'alcoxyde est choisi parmi le bis-butoxytriglycolate de magnésium, le bis-ω-méthoxypolyéthoxyde de magnésium, le bis-butoxytriglycolate de zinc, le bis-ω-méthoxypolyéthoxyde de zinc, le bis-hexylcarbitolate de magnésium, le bis-hexylcarbitolate de zinc, le ω-méthoxypolyéthoxyde d'éthoxymagnésium, l'éthoxytriglycolate de méthoxymagnésium, l'éthoxytriglycolate d'éthoxymagnésium, le butoxytriglycolate de méthoxymagnésium et le ω-méthoxypolyéthoxyde d'éthoxyzinc.